# EUROPÄISCHE PATENTSCHRIFT

(11) **EP 2 291 530 B1**
(45) Veröffentlichungstag und Bekanntmachung des Hinweises auf die Patenterteilung: **26.12.2018**
(21) Anmeldenummer: 09769063.0
(22) Anmeldetag: 28.04.2009
(51) Int. Cl.: C12P 7/42, C12P 7/62, C12N 15/63, C12N 15/70, C12N 9/10, C12N 9/90

(54) **2-HYDROXYISOBUTTERSÄURE PRODUZIERENDE REKOMBINANTE ZELLE**
RECOMBINANT CELL PRODUCING 2-HYDROXYISOBUTYRIC ACID
CELLULE RECOMBINANTE PRODUISANT DE L'ACIDE 2-HYDROXYISOBUTYRIQUE

(30) Priorität: 27.06.2008 DE 102008002715
(43) Veröffentlichungstag der Anmeldung: 09.03.2011
(73) Patentinhaber: Evonik Röhm GmbH, 64293 Darmstadt (DE)
(72) Erfinder: REINECKE, Liv, 45134 Essen (DE); SCHAFFER, Steffen, 45699 Herten (DE); MARX, Achim, 63571 Gelnhausen (DE); PÖTTER, Markus, 48149 Münster (DE); HAAS, Thomas, 48161 Münster (DE)
(86) Internationale Anmeldenummer: PCT/EP2009/055089
(87) Internationale Veröffentlichungsnummer: WO 2009/156214

(56) Entgegenhaltungen:
- WO-A-2007/110394
- REN Q ET AL: "PROPERTIES OF ENGINEERED POLY-3-HYDROXYALKANOATES PRODUCED IN RECOMBINANT ESCHERICHIA COLI STRAINS" APPLIED AND ENVIRONMENTAL MICROBIOLOGY, AMERICAN SOCIETY FOR MICROBIOLOGY, US, Bd. 66, Nr. 4, 1. April 2000 (2000-04-01), Seiten 1311-1320, XP000925369 ISSN: 0099-2240
- FUKUI TOSHIAKI ET AL: "Engineering of Ralstonia eutropha for production of poly(3-hydroxybutyrate-co-3-hydroxyhexanoa te) from fructose and solid-state properties of the copolymer" BIOMACROMOLECULES, Bd. 3, Nr. 3, Mai 2002 (2002-05), Seiten 618-624, XP002538853 ISSN: 1525-7797
- HOLOWACH L P ET AL: "Bacterial conversion of a waste stream containing methyl-2-hydroxyisobutyric acid to biodegradable polyhydroxyalkanoate polymers" WATER-SOLUBLE POLYMERS: SYNTHESIS, SOLUTION PROPERTIES AND APPLICATIONS, AMERICAN CHEMICAL SOCIETY, WASHINGTON, DC, US, Bd. 575, 1. Januar 1994 (1994-01-01), Seiten 202-211, XP002957523 ISBN: 978-0-541-23408-9
- ROHWERDER T ET AL: "The alkyl tert-butyl ether intermediate 2-hydroxyisobutyrate Is degraded via a novel cobalamin-dependent mutase pathway" APPLIED AND ENVIRONMENTAL MICROBIOLOGY JUNE 2006 AMERICAN SOCIETY FOR MICROBIOLOGY US, Bd. 72, Nr. 6, Juni 2006 (2006-06), Seiten 4128-4135, XP002460829

### SEQUENCE LISTING

<110> Evonik Röhm GmbH
<120> 2-Hydroxyisobuttersäure produzierende rekombinante Zelle
<130> 2008P00128
<160> 2
<170> PatentIn version 3.4
<210> 1
   <211> 7870
   <212> DNA
   <213> Artificial
<220>
   <223> Plasmid
<400> 1
   caaggagatg gcgcccaaca gtcccccggc cacggggcct gccaccatac ccacgccgaa      60
   acaagcgctc atgagcccga agtggcgagc ccgatcttcc ccatcggtga tgtcggcgat     120
   ataggcgcca gcaaccgcac ctgtggcgcc ggtgatgccg gccacgatgc gtccggcgta     180
   gaggatcgag atctcgatcc cgcgaaatta atacgactca ctatagggga attgtgagcg     240
   gataacaatt cccctctaga aataattttg tttaacttta agaaggaatt caggagccct     300
   tcaccatgac ctggcttgag ccgcagataa agtcccaact ccaatcggag cgcaaggact     360
   gggaagcgaa cgaagtcggc gccttcttga agaaggcccc cgagcgcaag gagcagttcc     420
   acacgatcgg ggacttcccg gtccagcgca cctacaccgc tgccgacatc gccgacacgc     480
   cgctggagga catcggtctt ccggggcgct acccgttcac gcgcgggccc tacccgacga     540
   tgtaccgcag ccgcacctgg acgatgcgcc agatcgccgg cttcggcacc ggcgaggaca     600
   ccaacaagcg cttcaagtat ctgatcgcgc agggccagac cggcatctcc accgacttcg     660
   acatgcccac gctgatgggc tacgactccg accacccgat gagcgacggc gaggtcggcc     720
   gcgagggcgt ggcgatcgac acgctggccg acatggaggc gctgctggcc gacatcgacc     780
   tcgagaagat ctcggtctcg ttcacgatca acccgagcgc ctggatcctg ctcgcgatgt     840
   acgtggcgct cggcgagaag cgcggctacg acctgaacaa gctgtcgggc acggtgcagg     900
   ccgacatcct gaaggagtac atggcgcaga aggagtacat ctacccgatc gcgccgtcgg     960
   tgcgcatcgt gcgcgacatc atcacctaca gcgcgaagaa cctgaagcgc tacaacccga    1020
   tcaacatctc gggctaccac atcagcgagg ccggctcctc gccgctccag gaggcggcct    1080
   tcacgctggc caacctgatc acctacgtga acgaggtgac gaagaccggt atgcacgtcg    1140
   acgaattcgc gccgcggttg gccttcttct tcgtgtcgca aggtgacttc ttcgaggagg    1200
   tcgcgaagtt ccgcgccctg cgccgctgct acgcgaagat catgaaggag cgcttcggtg    1260
   caagaaatcc ggaatcgatg cggttgcgct tccactgtca gaccgcggcg gcgacgctga    1320
   ccaagccgca gtacatggtc aacgtcgtgc gtacgtcgct gcaggcgctg tcggccgtgc    1380
   tcggcggcgc gcagtcgctg cacaccaacg gctacgacga agccttcgcg atcccgaccg    1440
   aggatgcgat gaagatggcg ctgcgcacgc agcagatcat tgccgaggag agtggtgtcg 1500
   ccgacgtgat cgacccgctg ggtggcagct actacgtcga ggcgctgacc accgagtacg 1560
   agaagaagat cttcgagatc ctcgaggaag tcgagaagcg cggtggcacc atcaagctga 1620
   tcgagcaggg ctggttccag aagcagattg cggacttcgc ttacgagacc gcgctgcgca 1680
   agcagtccgg ccagaagccg gtgatcgggg tgaaccgctt cgtcgagaac gaagaggacg 1740
   tcaagatcga gatccacccg tacgacaaca cgacggccga acgccagatt tcccgcacgc 1800
   gccgcgttcg cgccgagcgc gacgaggcca aggtgcaagc gatgctcgac caactggtgg 1860
   ctgtcgccaa ggacgagtcc cagaacctga tgccgctgac catcgaactg gtgaaggccg 1920
   gcgcaacgat gggggacatc gtcgagaagc tgaaggggat ctggggtacc taccgcgaga 1980
   cgccggtctt ctgagcagga agcttcccac catggaccaa atcccgatcc gcgttcttct 2040
   cgccaaagtc ggcctcgacg gccatgaccg aggcgtcaag gtcgtcgctc gcgcgctgcg 2100
   cgacgccggc atggacgtca tctactccgg ccttcatcgc acgcccgaag aggtggtcaa 2160
   caccgccatc caggaagacg tggacgtgct gggtgtaagc ctcctgtccg gcgtgcagct 2220
   cacggtcttc cccaagatct tcaagctcct ggacgagaga ggcgctggcg acttgatcgt 2280
   gatcgccggt ggcgtgatgc cggacgagga cgccgcggcc atccgcaagc tcggcgtgcg 2340
   cgaggtgcta ctgcaggaca cgcccccgca ggccatcatc gactcgatcc gctccttggt 2400
   cgccgcgcgc ggcgcccgct gaaagggcga gctcaattcg aagcttgaag gtaagcctat 2460
   ccctaaccct ctcctcggtc tcgattctac gcgtaccggt catcatcacc atcaccattg 2520
   agtttgatcc ggctgctaac aaagcccgaa aggaagctga gttggctgct gccaccgctg 2580
   agcaataact agcataaccc cttggggcct ctaaacgggt cttgaggggt tttttgctga 2640
   aaggaggaac tatatccgga tatcccgcaa gaggcccggc agtaccggca taaccaagcc 2700
   tatgcctaca gcatccaggg tgacggtgcc gaggatgacg atgagcgcat tgttagattt 2760
   catacacggt gcctgactgc gttagcaatt taactgtgat aaactaccgc attaaagctt 2820
   atcgatgata agctgtcaaa catgagaatt aattcttgaa gacgaaaggg cctcgtgata 2880
   cgcctatttt tataggttaa tgtcatgata ataatggttt cttagacgtc aggtggcact 2940
   tttcggggaa atgtgcgcgg aacccctatt tgtttatttt tctaaataca ttcaaatatg 3000
   tatccgctca tgagacaata accctgataa atgcttcaat aatattgaaa aaggaagagt 3060
   atgagtattc aacatttccg tgtcgccctt attccctttt ttgcggcatt ttgccttcct 3120
   gtttttgctc acccagaaac gctggtgaaa gtaaaagatg ctgaagatca gttgggtgca 3180
   cgagtgggtt acatcgaact ggatctcaac agcggtaaga tccttgagag ttttcgcccc 3240
   gaagaacgtt ttccaatgat gagcactttt aaagttctgc tatgtggcgc ggtattatcc 3300
   cgtgttgacg ccgggcaaga gcaactcggt cgccgcatac actattctca gaatgacttg 3360
   gttgagtact caccagtcac agaaaagcat cttacggatg gcatgacagt aagagaatta 3420
   tgcagtgctg ccataaccat gagtgataac actgcggcca acttacttct gacaacgatc 3480
   ggaggaccga aggagctaac cgcttttttg cacaacatgg gggatcatgt aactcgcctt 3540
   gatcgttggg aaccggagct gaatgaagcc ataccaaacg acgagcgtga caccacgatg 3600
   cctgcagcaa tggcaacaac gttgcgcaaa ctattaactg gcgaactact tactctagct 3660
   tcccggcaac aattaataga ctggatggag gcggataaag ttgcaggacc acttctgcgc 3720
   tcggcccttc cggctggctg gtttattgct gataaatctg gagccggtga gcgtgggtct 3780
   cgcggtatca ttgcagcact ggggccagat ggtaagccct cccgtatcgt agttatctac 3840
   acgacgggga gtcaggcaac tatggatgaa cgaaatagac agatcgctga gataggtgcc 3900
   tcactgatta agcattggta actgtcagac caagtttact catatatact ttagattgat 3960
   ttaaaacttc atttttaatt taaaaggatc taggtgaaga tcctttttga taatctcatg 4020
   accaaaatcc cttaacgtga gttttcgttc cactgagcgt cagaccccgt agaaaagatc 4080
   aaaggatctt cttgagatcc tttttttctg cgcgtaatct gctgcttgca aacaaaaaaa 4140
   ccaccgctac cagcggtggt ttgtttgccg gatcaagagc taccaactct ttttccgaag 4200
   gtaactggct tcagcagagc gcagatacca aatactgtcc ttctagtgta gccgtagtta 4260
   ggccaccact tcaagaactc tgtagcaccg cctacatacc tcgctctgct aatcctgtta 4320
   ccagtggctg ctgccagtgg cgataagtcg tgtcttaccg ggttggactc aagacgatag 4380
   ttaccggata aggcgcagcg gtcgggctga acggggggtt cgtgcacaca gcccagcttg 4440
   gagcgaacga cctacaccga actgagatac ctacagcgtg agctatgaga aagcgccacg 4500
   cttcccgaag ggagaaaggc ggacaggtat ccggtaagcg gcagggtcgg aacaggagag 4560
   cgcacgaggg agcttccagg gggaaacgcc tggtatcttt atagtcctgt cgggtttcgc 4620
   cacctctgac ttgagcgtcg atttttgtga tgctcgtcag gggggcggag cctatggaaa 4680
   aacgccagca acgcggcctt tttacggttc ctggcctttt gctggccttt tgctcacatg 4740
   ttctttcctg cgttatcccc tgattctgtg gataaccgta ttaccgcctt tgagtgagct 4800
   gataccgctc gccgcagccg aacgaccgag cgcagcgagt cagtgagcga ggaagcggaa 4860
   gagcgcctga tgcggtattt tctccttacg catctgtgcg gtatttcaca ccgcaatggt 4920
   gcactctcag tacaatctgc tctgatgccg catagttaag ccagtataca ctccgctatc 4980
   gctacgtgac tgggtcatgg ctgcgccccg acacccgcca acacccgctg acgcgccctg 5040
   acgggcttgt ctgctcccgg catccgctta cagacaagct gtgaccgtct ccgggagctg 5100
   catgtgtcag aggttttcac cgtcatcacc gaaacgcgcg aggcagctgc ggtaaagctc 5160
   atcagcgtgg tcgtgaagcg attcacagat gtctgcctgt tcatccgcgt ccagctcgtt 5220
   gagtttctcc agaagcgtta atgtctggct tctgataaag cgggccatgt taagggcggt 5280
   tttttcctgt ttggtcactg atgcctccgt gtaaggggga tttctgttca tgggggtaat 5340
   gataccgatg aaacgagaga ggatgctcac gatacgggtt actgatgatg aacatgcccg 5400
   gttactggaa cgttgtgagg gtaaacaact ggcggtatgg atgcggcggg accagagaaa 5460
   aatcactcag ggtcaatgcc agcgcttcgt taatacagat gtaggtgttc cacagggtag 5520
   ccagcagcat cctgcgatgc agatccggaa cataatggtg cagggcgctg acttccgcgt   5580
   ttccagactt tacgaaacac ggaaaccgaa gaccattcat gttgttgctc aggtcgcaga   5640
   cgttttgcag cagcagtcgc ttcacgttcg ctcgcgtatc ggtgattcat tctgctaacc   5700
   agtaaggcaa ccccgccagc ctagccgggt cctcaacgac aggagcacga tcatgcgcac   5760
   ccgtggccag gacccaacgc tgcccgagat gcgccgcgtg cggctgctgg agatggcgga   5820
   cgcgatggat atgttctgcc aagggttggt ttgcgcattc acagttctcc gcaagaattg   5880
   attggctcca attcttggag tggtgaatcc gttagcgagg tgccgccggc ttccattcag   5940
   gtcgaggtgg cccggctcca tgcaccgcga cgcaacgcgg ggaggcagac aaggtatagg   6000
   gcggcgccta caatccatgc caacccgttc catgtgctcg ccgaggcggc ataaatcgcc   6060
   gtgacgatca gcggtccaat gatcgaagtt aggctggtaa gagccgcgag cgatccttga   6120
   agctgtccct gatggtcgtc atctacctgc ctggacagca tggcctgcaa cgcgggcatc   6180
   ccgatgccgc cggaagcgag aagaatcata atggggaagg ccatccagcc tcgcgtcgcg   6240
   aacgccagca agacgtagcc cagcgcgtcg gccgccatgc cggcgataat ggcctgcttc   6300
   tcgccgaaac gtttggtggc gggaccagtg acgaaggctt gagcgagggc gtgcaagatt   6360
   ccgaataccg caagcgacag gccgatcatc gtcgcgctcc agcgaaagcg gtcctcgccg   6420
   aaaatgaccc agagcgctgc cggcacctgt cctacgagtt gcatgataaa gaagacagtc   6480
   ataagtgcgg cgacgatagt catgccccgc gcccaccgga aggagctgac tgggttgaag   6540
   gctctcaagg gcatcggtcg agatcccggt gcctaatgag tgagctaact tacattaatt   6600
   gcgttgcgct cactgcccgc tttccagtcg ggaaacctgt cgtgccagct gcattaatga   6660
   atcggccaac gcgcggggag aggcggtttg cgtattgggc gccagggtgg tttttctttt   6720
   caccagtgag acgggcaaca gctgattgcc cttcaccgcc tggccctgag agagttgcag   6780
   caagcggtcc acgctggttt gccccagcag gcgaaaatcc tgtttgatgg tggttaacgg   6840
   cgggatataa catgagctgt cttcggtatc gtcgtatccc actaccgaga tatccgcacc   6900
   aacgcgcagc ccggactcgg taatggcgcg cattgcgccc agcgccatct gatcgttggc   6960
   aaccagcatc gcagtgggaa cgatgccctc attcagcatt tgcatggttt gttgaaaacc   7020
   ggacatggca ctccagtcgc cttcccgttc cgctatcggc tgaatttgat tgcgagtgag   7080
   atatttatgc cagccagcca gacgcagacg cgccgagaca gaacttaatg ggcccgctaa   7140
   cagcgcgatt tgctggtgac ccaatgcgac cagatgctcc acgcccagtc gcgtaccgtc   7200
   ttcatgggag aaaataatac tgttgatggg tgtctggtca gagacatcaa gaaataacgc   7260
   cggaacatta gtgcaggcag cttccacagc aatggcatcc tggtcatcca gcggatagtt   7320
   aatgatcagc ccactgacgc gttgcgcgag aagattgtgc accgccgctt tacaggcttc   7380
   gacgccgctt cgttctacca tcgacaccac cacgctggca cccagttgat cggcgcgaga   7440
   tttaatcgcc gcgacaattt gcgacggcgc gtgcagggcc agactggagg tggcaacgcc   7500
   aatcagcaac gactgtttgc ccgccagttg ttgtgccacg cggttgggaa tgtaattcag   7560
   ctccgccatc gccgcttcca ctttttcccg cgttttcgca gaaacgtggc tggcctggtt 7620
   caccacgcgg gaaacggtct gataagagac accggcatac tctgcgacat cgtataacgt 7680
   tactggtttc acattcacca ccctgaattg actctcttcc gggcgctatc atgccatacc 7740
   gcgaaaggtt ttgcgccatt cgatggtgtc cgggatctcg acgctctccc ttatgcgact 7800
   cctgcattag gaagcagccc agtagtaggt tgaggccgtt gagcaccgcc gccgcaagga 7860
   atggtgcatg 7870
<210> 2
   <211> 7296
   <212> DNA
   <213> Artificial
<220>
   <223> Plasmid
<400> 2
   accttcggga gcgcctgaag cccgttctgg acgccctggg gccgttgaat cgggatatgc     60
   aggccaaggc cgccgcgatc atcaaggccg tgggcgaaaa gctgctgacg gaacagcggg    120
   aagtccagcg ccagaaacag gcccagcgcc agcaggaacg cgggcgcgca catttccccg    180
   aaaagtgcca cctggatgaa atgtcagcta ctgggctatc tggacaaggg aaaacgcaag    240
   cgcaaagaga aagcaggtag cttgcagtgg gcttacatgg cgatagctag actgggcggt    300
   tttatggaca gcaagcgaac cggaattgcc agctggggcg ccctctggta aggttgggaa    360
   gccctgcaaa gtaaactgga tggctttctt gccgccaagg atctgatggc gcaggggatc    420
   aagatctgat caagagacag gatgaggatc gtttcgcatg attgaacaag atggattgca    480
   cgcaggttct ccggccgctt gggtggagag gctattcggc tatgactggg cacaacagac    540
   aatcggctgc tctgatgccg ccgtgttccg gctgtcagcg caggggcgcc cggttctttt    600
   tgtcaagacc gacctgtccg gtgccctgaa tgaactgcag gacgaggcag cgcggctatc    660
   gtggctggcc acgacgggcg ttccttgcgc agctgtgctc gacgttgtca ctgaagcggg    720
   aagggactgg ctgctattgg gcgaagtgcc ggggcaggat ctcctgtcat ctcaccttgc    780
   tcctgccgag aaagtatcca tcatggctga tgcaatgcgg cggctgcata cgcttgatcc    840
   ggctacctgc ccattcgacc accaagcgaa acatcgcatc gagcgagcac gtactcggat    900
   ggaagccggt cttgtcgatc aggatgatct ggacgaagag catcaggggc tcgcgccagc    960
   cgaactgttc gccaggctca aggcgcgcat gcccgacggc gaggatctcg tcgtgaccca   1020
   tggcgatgcc tgcttgccga atatcatggt ggaaaatggc cgcttttctg gattcatcga   1080
   ctgtggccgg ctgggtgtgg cggaccgcta tcaggacata gcgttggcta cccgtgatat   1140
   tgctgaagag cttggcggcg aatgggctga ccgcttcctc gtgctttacg gtatcgccgc   1200
   tcccgattcg cagcgcatcg ccttctatcg ccttcttgac gagttcttct gagcgggact   1260
   ctggggttcg aaatgaccga ccaagcgacg cccaacctgc catcacgaga tttcgattcc   1320
   accgccgcct tctatgaaag gttgggcttc ggaatcgttt tccgggacgc cggctggatg   1380
   atcctccagc gcggggatct catgctggag ttcttcgccc acccccatgg gcaaatatta 1440
   tacgcaaggc gacaaggtgc tgatgccgct ggcgattcag gttcatcatg ccgtttgtga 1500
   tggcttccat gtcggcagaa tgcttaatga attacaacag tttttatgca tgcgcccaat 1560
   acgcaaaccg cctctccccg cgcgttggcc gattcattaa tgcagctggc acgacaggtt 1620
   tcccgactgg aaagcgggca gtgagcgcaa cgcaattaat gtgagttagc tcactcatta 1680
   ggcaccccag gctttacact ttatgcttcc ggctcgtatg ttgtgtggaa ttgtgagcgg 1740
   ataacaattt cacacaggaa acagctatga ccatgattac gccaagcgcg caattaaccc 1800
   tcactaaagg gaacaaaagc tgggtaccgg gccccccctc gaggtcgacg gtatcgataa 1860
   gcttgatatc gaattcctgc agcccggggg atccactagt tctagaaata attttgttta 1920
   actttaagaa ggaattcagg agcccttcac catgacctgg cttgagccgc agataaagtc 1980
   ccaactccaa tcggagcgca aggactggga agcgaacgaa gtcggcgcct tcttgaagaa 2040
   ggcccccgag cgcaaggagc agttccacac gatcggggac ttcccggtcc agcgcaccta 2100
   caccgctgcc gacatcgccg acacgccgct ggaggacatc ggtcttccgg ggcgctaccc 2160
   gttcacgcgc gggccctacc cgacgatgta ccgcagccgc acctggacga tgcgccagat 2220
   cgccggcttc ggcaccggcg aggacaccaa caagcgcttc aagtatctga tcgcgcaggg 2280
   ccagaccggc atctccaccg acttcgacat gcccacgctg atgggctacg actccgacca 2340
   cccgatgagc gacggcgagg tcggccgcga gggcgtggcg atcgacacgc tggccgacat 2400
   ggaggcgctg ctggccgaca tcgacctcga gaagatctcg gtctcgttca cgatcaaccc 2460
   gagcgcctgg atcctgctcg cgatgtacgt ggcgctcggc gagaagcgcg gctacgacct 2520
   gaacaagctg tcgggcacgg tgcaggccga catcctgaag gagtacatgg cgcagaagga 2580
   gtacatctac ccgatcgcgc cgtcggtgcg catcgtgcgc gacatcatca cctacagcgc 2640
   gaagaacctg aagcgctaca acccgatcaa catctcgggc taccacatca gcgaggccgg 2700
   ctcctcgccg ctccaggagg cggccttcac gctggccaac ctgatcacct acgtgaacga 2760
   ggtgacgaag accggtatgc acgtcgacga attcgcgccg cggttggcct tcttcttcgt 2820
   gtcgcaaggt gacttcttcg aggaggtcgc gaagttccgc gccctgcgcc gctgctacgc 2880
   gaagatcatg aaggagcgct tcggtgcaag aaatccggaa tcgatgcggt tgcgcttcca 2940
   ctgtcagacc gcggcggcga cgctgaccaa gccgcagtac atggtcaacg tcgtgcgtac 3000
   gtcgctgcag gcgctgtcgg ccgtgctcgg cggcgcgcag tcgctgcaca ccaacggcta 3060
   cgacgaagcc ttcgcgatcc cgaccgagga tgcgatgaag atggcgctgc gcacgcagca 3120
   gatcattgcc gaggagagtg gtgtcgccga cgtgatcgac ccgctgggtg gcagctacta 3180
   cgtcgaggcg ctgaccaccg agtacgagaa gaagatcttc gagatcctcg aggaagtcga 3240
   gaagcgcggt ggcaccatca agctgatcga gcagggctgg ttccagaagc agattgcgga 3300
   cttcgcttac gagaccgcgc tgcgcaagca gtccggccag aagccggtga tcggggtgaa 3360
   ccgcttcgtc gagaacgaag aggacgtcaa gatcgagatc cacccgtacg acaacacgac 3420
   ggccgaacgc cagatttccc gcacgcgccg cgttcgcgcc gagcgcgacg aggccaaggt   3480
   gcaagcgatg ctcgaccaac tggtggctgt cgccaaggac gagtcccaga acctgatgcc   3540
   gctgaccatc gaactggtga aggccggcgc aacgatgggg gacatcgtcg agaagctgaa   3600
   ggggatctgg ggtacctacc gcgagacgcc ggtcttctga gcaggaagct tcccaccatg   3660
   gaccaaatcc cgatccgcgt tcttctcgcc aaagtcggcc tcgacggcca tgaccgaggc   3720
   gtcaaggtcg tcgctcgcgc gctgcgcgac gccggcatgg acgtcatcta ctccggcctt   3780
   catcgcacgc ccgaagaggt ggtcaacacc gccatccagg aagacgtgga cgtgctgggt   3840
   gtaagcctcc tgtccggcgt gcagctcacg gtcttcccca agatcttcaa gctcctggac   3900
   gagagaggcg ctggcgactt gatcgtgatc gccggtggcg tgatgccgga cgaggacgcc   3960
   gcggccatcc gcaagctcgg cgtgcgcgag gtgctactgc aggacacgcc cccgcaggcc   4020
   atcatcgact cgatccgctc cttggtcgcc gcgcgcggcg cccgctgaaa gggcgagctc   4080
   tccaattcgc cctatagtga gtcgtattac gcgcgctcac tggccgtcgt tttacaacgt   4140
   cgtgactggg aaaaccctgg cgttacccaa cttaatcgcc ttgcagcaca tccccctttc   4200
   gccagctggc gtaatagcga agaggcccgc accgatcgcc cttcccaaca gttgcgcagc   4260
   ctgaatggcg aatggaaatt gtaagcgtta atattttgtt aaaattcgcg ttaaattttt   4320
   gttaaatcag ctcatttttt aaccaatagg ccgactgcga tgagtggcag ggcggggcgt   4380
   aattttttta aggcagttat tggtgccctt aaacgcctgg tgctacgcct gaataagtga   4440
   taataagcgg atgaatggca gaaattcgaa agcaaattcg acccggtcgt cggttcaggg   4500
   cagggtcgtt aaatagccgc ttatgtctat tgctggttta ccggtttatt gactaccgga   4560
   agcagtgtga ccgtgtgctt ctcaaatgcc tgaggccagt ttgctcaggc tctccccgtg   4620
   gaggtaataa ttgacgatat gatcatttat tctgcctccc agagcctgat aaaaacggtg   4680
   aatccgttag cgaggtgccg ccggcttcca ttcaggtcga ggtggcccgg ctccatgcac   4740
   cgcgacgcaa cgcggggagg cagacaaggt atagggcggc gaggcggcta cagccgatag   4800
   tctggaacag cgcacttacg ggttgctgcg caacccaagt gctaccggcg cggcagcgtg   4860
   acccgtgtcg gcggctccaa cggctcgcca tcgtccagaa aacacggctc atcgggcatc   4920
   ggcaggcgct gctgcccgcg ccgttcccat tcctccgttt cggtcaaggc tggcaggtct   4980
   ggttccatgc ccggaatgcc gggctggctg ggcggctcct cgccggggcc ggtcggtagt   5040
   tgctgctcgc ccggatacag ggtcgggatg cggcgcaggt cgccatgccc caacagcgat   5100
   tcgtcctggt cgtcgtgatc aaccaccacg gcggcactga acaccgacag gcgcaactgg   5160
   tcgcggggct ggccccacgc cacgcggtca ttgaccacgt aggccgacac ggtgccgggg   5220
   ccgttgagct tcacgacgga gatccagcgc tcggccacca agtccttgac tgcgtattgg   5280
   accgtccgca aagaacgtcc gatgagcttg gaaagtgtct tctggctgac caccacggcg   5340
   ttctggtggc ccatctgcgc cacgaggtga tgcagcagca ttgccgccgt gggtttcctc   5400
   gcaataagcc cggcccacgc ctcatgcgct ttgcgttccg tttgcaccca gtgaccgggc   5460
   ttgttcttgg cttgaatgcc gatttctctg gactgcgtgg ccatgcttat ctccatgcgg   5520
   tagggtgccg cacggttgcg gcaccatgcg caatcagctg caacttttcg gcagcgcgac   5580
   aacaattatg cgttgcgtaa aagtggcagt caattacaga ttttctttaa cctacgcaat   5640
   gagctattgc ggggggtgcc gcaatgagct gttgcgtacc cccctttttt aagttgttga   5700
   tttttaagtc tttcgcattt cgccctatat ctagttcttt ggtgcccaaa gaagggcacc   5760
   cctgcggggt tcccccacgc cttcggcgcg gctccccctc cggcaaaaag tggcccctcc   5820
   ggggcttgtt gatcgactgc gcggccttcg gccttgccca aggtggcgct gcccccttgg   5880
   aacccccgca ctcgccgccg tgaggctcgg ggggcaggcg ggcgggcttc gccttcgact   5940
   gcccccactc gcataggctt gggtcgttcc aggcgcgtca aggccaagcc gctgcgcggt   6000
   cgctgcgcga gccttgaccc gccttccact tggtgtccaa ccggcaagcg aagcgcgcag   6060
   gccgcaggcc ggaggctttt ccccagagaa aattaaaaaa attgatgggg caaggccgca   6120
   ggccgcgcag ttggagccgg tgggtatgtg gtcgaaggct gggtagccgg tgggcaatcc   6180
   ctgtggtcaa gctcgtgggc aggcgcagcc tgtccatcag cttgtccagc agggttgtcc   6240
   acgggccgag cgaagcgagc cagccggtgg ccgctcgcgg ccatcgtcca catatccacg   6300
   ggctggcaag ggagcgcagc gaccgcgcag ggcgaagccc ggagagcaag cccgtagggc   6360
   gccgcagccg ccgtaggcgg tcacgacttt gcgaagcaaa gtctagtgag tatactcaag   6420
   cattgagtgg cccgccggag gcaccgcctt gcgctgcccc cgtcgagccg gttggacacc   6480
   aaaagggagg ggcaggcatg gcggcatacg cgatcatgcg atgcaagaag ctggcgaaaa   6540
   tgggcaacgt ggcggccagt ctcaagcacg cctaccgcga gcgcgagacg cccaacgctg   6600
   acgccagcag gacgccagag aacgagcact gggcggccag cagcaccgat gaagcgatgg   6660
   gccgactgcg cgagttgctg ccagagaagc ggcgcaagga cgctgtgttg gcggtcgagt   6720
   acgtcatgac ggccagcccg gaatggtgga agtcggccag ccaagaacag caggcggcgt   6780
   tcttcgagaa ggcgcacaag tggctggcgg acaagtacgg ggcggatcgc atcgtgacgg   6840
   ccagcatcca ccgtgacgaa accagcccgc acatgaccgc gttcgtggtg ccgctgacgc   6900
   aggacggcag gctgtcggcc aaggagttca tcggcaacaa agcgcagatg acccgcgacc   6960
   agaccacgtt tgcggccgct gtggccgatc tagggctgca acggggcatc gagggcagca   7020
   aggcacgtca cacgcgcatt caggcgttct acgaggccct ggagcggcca ccagtgggcc   7080
   acgtcaccat cagcccgcaa gcggtcgagc cacgcgccta tgcaccgcag ggattggccg   7140
   aaaagctggg aatctcaaag cgcgttgaga cgccggaagc cgtggccgac cggctgacaa   7200
   aagcggttcg gcaggggtat gagcctgccc tacaggccgc cgcaggagcg cgtgagatgc   7260
   gcaagaaggc cgatcaagcc caagagacgg cccgag                             7296

## Beschreibung

### Gebiet der Erfindung

Gegenstand der Erfindung ist eine Zelle, welche derart gentechnisch verändert wurde, dass sie im Vergleich zu ihrem Wildtyp mehr 2-Hydroxyisobuttersäure zu bilden vermag, dadurch gekennzeichnet, dass die Bildung von 2-Hydroxyisobuttersäure über Acetoacetyl-Coenzym A als Zwischenprodukt und 3-Hydroxybutyryl-Coenzym A als Vorprodukt erfolgt.

### Stand der Technik

Methacrylsäure, deren Ester und Polymere finden breite Anwendung bei der Produktion von Acrylglasscheiben, Spritzgussformprodukten, Beschichtungen und vielen anderen Produkten.

Mehrere Prozesse zur Herstellung von Methacrylsäure sind bekannt. Allerdings basiert der weltweit größte Teil der kommerziellen Produktion auf einem chemischen Verfahren zur Hydrolyse der Amid-Sulfate von Methacrylsäure, die aus den entsprechenden 2-Hydroxynitrilen produziert werden, wobei etwa 1 ,6 kg Schwefelsäure zur Produktion von 1 kg Methacrylsäure benötigt werden.

Die chemische Umwandlung von 2-Hydroxyisobuttersäure (2-HIB) in Methacrylsäure mit Ausbeuten von bis zu 96 % wird in US 3,666,805 and US 5,225,594 beschrieben.

Ein alternatives Verfahren zur Produktion von Methacrylsäure ergibt sich durch Hydrolyse von 2-Hydroxynitrilen zu 2-Hydroxyisobuttersäure unter Nutzung von Nitril-hydrolysierenden Enzymen. Dabei handelt es sich um Nitrilase oder eine Kombination aus Nitril-Hydratase und Amidase (A. Banerjee, R. Sharrna, U. C. Banerjee, 2002, "The nitrile-degrading enzymes: current status and future prospects", Appl. Microbiol. Biotechnol., 60:33- 44 und US 6,582,943). Ein schwerwiegender Nachteil dieser Methode ist die Instabilität der Nitrile im für eine effiziente Nitril-hydrolisierende Enzymaktivität benötigten neutralen pH-Bereich. Der Zerfall der Nitrile im Reaktionsgemisch führt zu einer Akkumulation von Ketonen und Cyanid, die beide die Nitril-hydrolisierenden Enzymaktivitäten hemmen.

Ein genereller Nachteil beider Verfahren, d.h. des zurzeit dominierenden Verfahrens auf Basis der Amid-Sulfate und des enzymatischen Nitril-hydrolysierenden Verfahrens, ist der Bedarf an 2-Hydroxynitrilen. Diese müssen erst aus umweltschädlichen Edukten, namentlich Ketone und Cyanid, hergestellt werden.

Ein alternatives Verfahren zur Bereitstellung von 2-Hydroxyisobuttersäure über einen enzymatischen, tert-Butylalkohol degradierenden Stoffwechselweg ist bekannt aus CA 2,510,657.

Ein weiteres enzymatisches Verfahren zur Bereitstellung von 2-Hydroxyisobuttersäure ist bekannt aus WO 2007/110394. Hier wird mit Hilfe einer Mutase 3-Hydroxybutyryl-Coenzym A (3-HBCoA) als Vorprodukt in 2-Hydroxyisobuttersäure umgewandelt. Das ausgeführte Verfahren weist folgende Nachteile auf: Das ausgeführte Verfahren ist diskontinuierlich, 3-Hydroxybuttersäure (3-HB) wird exogen als Edukt zugegeben und die Verfahrensbedingungen erfordern Inertgas. Die Umsatzraten liegen bei 20%.

Deshalb wären Verfahren zur Herstellung von 2-Hydroxyisobuttersäure oder 2-Hydroxyisobuttersäure-Monomereinheiten enthaltende Polyhydroxyalkanoate, welches die beschriebenen Nachteile überwindet, von Vorteil.

Aufgabe der Erfindung war es daher, ein Verfahren zur Bildung von 2-Hydroxyisobuttersäure bereitzustellen, welches den Bedarf an Vorprodukt für 2-Hydroxyisobuttersäure oder 2-Hydroxyisobuttersäure-Monomereinheiten enthaltende Polyhydroxyalkanoate gewährleistet, welche zu Methacrylsäure, deren Ester und Polymere weiterverarbeitet werden können.

### Beschreibung der Erfindung

Überraschenderweise wurde gefunden, dass die Bildung von 2-Hydroxyisobuttersäure oder von 2-Hydroxyisobuttersäure-Monomereinheiten enthaltenden Polyhydroxyalkanoaten über Acetoacetyl-Coenzym A als Zwischenprodukt und 3-Hydroxybutyryl-Coenzym A als Vorprodukt, einen Beitrag zur Lösung der vorstehend genannten Aufgaben leistet.

Der Begriff "Vorprodukt", wie er hierin verwendet wird, definiert eine chemische Verbindung, die durch Einsatz lediglich eines Enzymes auf enzymatischem Weg zu dem gewünschten Produkt umgesetzt werden kann, während der Begriff "Zwischenprodukt" eine chemische Verbindung definiert, die durch den Einsatz mindestens zweier Enzyme auf enzymatischem Weg zu dem gewünschten Produkt umgesetzt werden kann, wobei als "chemische Verbindung" solche mit oder ohne Coenzym A-Thioester-Funktionalisierung als gleichwertig betrachtet werden sollen und somit die Thioester bildenden bzw. -spaltenden Enzyme nicht mitgezählt werden.

Gegenstand der vorliegenden Erfindung ist daher eine Bakterienzelle, welche derart gentechnisch verändert wurde, dass sie im Vergleich zu ihrem Wildtyp mehr 2-Hydroxyisobuttersäure zu bilden vermag, dadurch gekennzeichnet, dass die Bildung von 2-Hydroxyisobuttersäure über Acetoacetyl-Coenzym A als Zwischenprodukt und 3-Hydroxybutyryl-Coenzym A als Vorprodukt erfolgt, wobei die Zelle mindestens eine Aktivität eines Enzyms E₃ aufweist, welches die Umsetzung von 3-Hydroxybutyryl-Coenzym A zu 2-Hydroxyisobutyryl-Coenzym A katalysiert,
wobei die Zelle eine im Vergleich zu ihrem Wildtyp erhöhte Aktivität des Enzyms E₃ aufweist,
wobei die Zelle als Wildtyp eine Aktivität eines Enzyms E₄ aufweist, welches die Umsetung von 3-HydroxybutyrylCoenzym A zu Polyhydroxybutyrat katalysiert
wobei die Zelle eine im Vergleich zu ihrem Wildtyp erniedrigte Aktivität des Enzyms E₄ aufweist und wobei das Enzym E₃ ausgewählt wird aus einer HydroxylIsobutyryl-CoA Mutase, einer Isobutyryl-CoA Mutase (EC 5.4.99.13) oder einer Methylmalonyl-CoA Mutase (EC 5.4.99.2), und
wobei das Enzym E₄ eine Polyhydroxyalkanoat Synthase ist.

Ein weiterer Gegenstand der Erfindung ist ein Verfahren zur Herstellung von 2-Hydroxyisobuttersäure mit erfindungsgemäßer Zelle sowie ein Verfahren zur Herstellung von Methacrylsäure oder Methacrylsäureestern sowie ein Verfahren zur Herstellung von Polymethacrylsäure oder Polymethacrylsäureestern.

Ein Vorteil der Erfindung ist, dass 2-Hydroxyisobuttersäure bzw. Methacrylsäure sowohl aus nachwachsenden Rohstoffen, beispielsweise aus Kohlenhydraten und/oder aus Glycerin hergestellt werden kann aber auch aus von fossilen Brennstoffen abgeleiteten Rohstoffen wie beispielsweise Methanol und somit die Probleme von schwankender Verfügbarkeit der fossilen Rohstoffe umgangen werden können.

Ein weiterer Vorteil der Erfindung ist, dass bei dem erfindungsgemäßen Verfahren mit wenig thermisch belastenden Schritten und generell mit weniger Verfahrensschritten die Methacrylsäure erhalten werden kann.

Noch ein weiterer Vorteil der Erfindung ist die Vermeidung vieler toxischer bzw. aggressiver Substanzen, wie sie in herkömmlichen, chemischen Verfahren zur Herstellung von 2-Hydroxyisobuttersäure anfallen.

Die Erfindung wird nachfolgend beispielhaft beschrieben, ohne dass die Erfindung auf diese beispielhaften Ausführungsformen beschränkt sein soll.

Alle angegebenen Prozent (%) sind, wenn nicht anders angegeben, Massenprozent.

Der Begriff "2-Hydroxyisobuttersäure", wie er hierin verwendet wird, beschreibt stets die entsprechende C₄-Carbonsäure in derjenigen Form, in der sie nach Bildung durch die entsprechenden Mikroorganismen in Abhängigkeit vom pH-Wert vorliegt. Der Begriff umfasst somit stets die reine Säureform (2-Hydroxyisobuttersäure), die reine Basenform (2-Hydroxyisobutyrat) sowie Mischungen aus protonierter und deprotonierter Form der Säure.

Weiterhin umfasst der Begriff "3-Hydroxybutyryl-Coenzym A" grundsätzlich sowohl das (R)- als auch das (S)-Stereoisomer, wobei das (R)-Stereoisomer besonders bevorzugt ist.

Die Formulierung "dass sie im Vergleich zu ihrem Wildtyp mehr 2-Hydroxyisobuttersäure oder 2-Hydroxyisobuttersäure-Monomereinheiten enthaltende Polyhydroxyalkanoate zu bilden vermag" betrifft auch den Fall, dass der Wildtyp der gentechnisch veränderten Zelle überhaupt keine 2-Hydroxyisobuttersäure oder keine 2-Hydroxyisobuttersäure-Monomereinheiten enthaltende Polyhydroxyalkanoate, zumindest aber keine nachweisbaren Mengen dieser Verbindungen zu bilden vermag, und erst nach der gentechnischen Veränderung nachweisbare Mengen dieser Komponenten gebildet werden können. Unter einem "Wildtyp" einer Zelle wird vorzugsweise eine Zelle bezeichnet, deren Genom in einem Zustand vorliegt, wie er natürlicherweise durch die Evolution entstanden ist. Der Begriff wird sowohl für die gesamte Zelle als auch für einzelne Gene verwendet. Unter den Begriff "Wildtyp" fallen daher insbesondere nicht solche Zellen bzw. solche Gene, deren Gensequenzen zumindest teilweise durch den Menschen mittels rekombinanter Verfahren verändert worden sind.

Aus der 2-Hydroxyisobuttersäure kann anschließend durch eine schonende Dehydratisierungsreaktion Methacrylsäure erhalten werden. Im Falle von 2-Hydroxyisobuttersäure-Monomereinheiten enthaltende Polyhydroxyalkanoate können die in den Zellen enthaltenen Grana, welche mit diesen Polyhydroxyalkanoaten gefüllt sind, isoliert und anschließend die Polymere unter Erhalt von 2-Hydroxyisobuttersäure gespalten werden, welches dann unter Erhalt von Methacrylsäure dehydratisiert werden kann.

Dabei ist es erfindungsgemäß bevorzugt, dass die gentechnisch veränderte Zelle derart gentechnisch verändert ist, dass sie in einem definierten Zeitintervall, vorzugsweise innerhalb von 2 Stunden, noch mehr bevorzugt innerhalb von 8 Stunden und am meisten bevorzugt innerhalb von 24 Stunden, mindestens zweimal, besonders bevorzugt mindestens 10mal, darüber hinaus bevorzugt mindestens 100mal, darüber hinaus noch mehr bevorzugt mindestens 1.000mal und am meisten bevorzugt mindestens 10.000mal mehr 2-Hydroxyisobuttersäure oder 2-Hydroxyisobuttersäure-Monomereinheiten enthaltende Polyhydroxyalkanoate bildet als der Wildtyp der Zelle. Die Zunahme der Produktbildung kann dabei beispielsweise dadurch bestimmt werden, dass die erfindungsgemäße Zelle und die Wildtyp-Zelle jeweils getrennt unter gleichen Bedingungen (gleiche Zelldichte, gleiches Nährmedium, gleiche Kulturbedingungen) für ein bestimmtes Zeitintervall in einem geeigneten Nährmedium kultiviert werden und anschließend die Menge an Zielprodukt (2-Hydroxyisobuttersäure oder 2-Hydroxyisobuttersäure-Monomereinheiten enthaltende Polyhydroxyalkanoate) im Zellüberstand im Falle von 2-Hydroxyisobuttersäure bzw. in den Zellen im Falle von 2-Hydroxyisobuttersäure-Monomereinheiten enthaltenden Polyhydroxyalkanoaten bestimmt wird.

Die erfindungsgemäßen Zellen sind Bakterien. Als Bakterien, die bei der Deutschen Sammlung von Mikroorganismen und Zellkulturen GmbH (DSMZ), Braunschweig, Deutschland, als Bakterien-Stämme hinterlegt sind. Erfindungsgemäß geeignete Bakterien gehören zu den Gattungen, die unter http://www.dsmz.de/species/bacteria.htm

Erfindungsgemäß bevorzugte Zellen sind diejenigen der Gattungen *Corynebacterium, Brevibacterium, Bacillus, Acinetobacter, Alcaligenes, Lactobacillus, Paracoccus, Lactococcus,* , *Escherichia, Zymomonas, Methylobacterium, Ralstonia, Pseudomonas, Rhodospirillum, Rhodobacter, Burkholderia, Clostridium und Cupriavidus,* wobei *Alcaligenes latus, Bacillus megaterium, Bacillus subtilis, Brevibacterium flavum, Brevibacterium lactofermentum, Escherichia coli, Candida blankii, Candida rugosa, Corynebacterium glutamicum, Corynebacterium efficiens, Zymomonas mobilis, Methylobacterium extorquens, Ralstonia eutropha, insbesondere Ralstonia eutropha H16, Rhodospirillum rubrum, Rhodobacter sphaeroides, Paracoccus versutus, Pseudomonas aeruginosa, Pseudomonas putida, Acinetobacter calcoaceticus* und *Pichia pastoris* besonders bevorzugt sind.

Die erfindungsgemäße Bakterienzelle, die im Vergleich zu ihrem Wildtyp mehr 2-Hydroxyisobuttersäure oder 2-Hydroxyisobuttersäure-Monomereinheiten enthaltende Polyhydroxyalkanoate über Acetoacetyl-Coenzym A als Zwischenprodukt und 3-Hydroxybutyryl-Coenzym A als Vorprodukt zu bilden vermag, weist daher eine
Aktivität eines Enzyms E₁ auf, welches die Umsetzung von Acetoacetyl-Coenzym A zu 3-Hydroxybutyryl-Coenzym A katalysiert.

Bei dem Enzym E₁ handelt es sich vorzugsweise um ein Enzym ausgewählt aus der Gruppe enthaltend:
eine 3-Hydroxyacyl-CoA Dehydrogenase (EC 1.1.1.35),
eine Acetoacetyl-Coenzym A Reduktase (EC 1.1.1.36),
eine Long-Chain-3-Hydroxyacyl-CoA Dehydrogenase ((EC 1.1.1.211) und
eine 3-Hydroxybutyryl-Coenzym A-Dehydrogenase (EC 1.1.1.157).

Dieses Enzym wird vorzugsweise von den Genen kodiert ausgewählt aus der Gruppe bestehend aus phaB, phbB, fabG, phbN1, phbB2 oder, wobei phaB, phbB besonders bevorzugt sind. Die Nukleotidsequenz dieser Gene können beispielsweise der "Kyoto Encyclopedia of Genes and Genomes" (KEGG-Datenbank), den Datenbanken des National Center for Biotechnology Information (NCBI) der National Library of Medicine (Bethesda, MD, USA) oder der Nukleotidsequenz-Datenbank der European Molecular Biologies Laboratories (EMBL, Heidelberg, Deutschland bzw. Cambridge, UK) entnommen werden.

Es kann für die erfindungsgemäße Bakterienzelle vorteilhaft sein, dass die erfindungsgemäße Zelle im Vergleich zu ihrem Wildtyp eine gesteigerte Aktivität des Enzyms E₁ aufweist, welches die Umsetzung von Acetoacetyl-Coenzym A zu 3-Hydroxybutyryl-Coenzym A katalysiert.

Der Begriff "gesteigerte Aktivität eines Enzyms", wie er vorstehend im Zusammenhang mit dem Enzym E₁ und in den nachfolgenden Ausführungen im Zusammenhang mit den Enzymen E₂ usw. verwendet wird, ist vorzugsweise als gesteigerte intrazelluläre Aktivität zu verstehen.

Die nun folgenden Ausführungen zur Erhöhung der Enzymaktivität in Zellen gelten sowohl für die Erhöhung der Aktivität des Enzyms E₁ als auch für alle nachfolgend genannten Enzyme, deren Aktivität gegebenenfalls erhöht werden kann.

Grundsätzlich lässt sich eine Steigerung der enzymatischen Aktivität dadurch erzielen, dass man die Kopienzahl der Gensequenz bzw. der Gensequenzen erhöht, welche für das Enzym kodieren, einen starken Promotor verwendet, die Kodonnutzung des Gens verändert, auf verschiedene Art und Weise die Halbwertszeit der mRNA oder des Enzyms erhöht, die Regulation der Expression des Gens modifiziert oder ein Gen oder Allel nutzt, das für ein entsprechendes Enzym mit einer gesteigerten Aktivität kodiert und gegebenenfalls diese Maßnahmen kombiniert. Erfindungsgemäß gentechnisch veränderte Zellen werden beispielsweise durch Transformation, Transduktion, Konjugation oder eine Kombination dieser Methoden mit einem Vektor erzeugt, der das gewünschte Gen, ein Allel dieses Gens oder Teile davon und einen die Expression des Gens ermöglichenden Promotor enthält. Die heterologe Expression wird insbesondere durch Integration des Gens oder der Allele in das Chromosom der Zelle oder einem extrachromosomal replizierenden Vektor erzielt.

Einen Überblick über die Möglichkeiten zur Erhöhung der EnzymAktivität in Zellen am Beispiel der Pyruvat-Carboxylase gibt DE-A-100 31 999.

Die Expression der vorstehend und aller nachfolgend genannten Enzyme bzw. Gene ist mit Hilfe von 1- und 2-dimensionaler

Proteingelauftrennung und anschließender optischer Identifizierung der Proteinkonzentration mit entsprechender Auswertesoftware im Gel nachweisbar. Wenn die Erhöhung einer Enzymaktivität ausschließlich auf einer Erhöhung der Expression des entsprechenden Gens basiert, so kann die Quantifizierung der Erhöhung der Enzymaktivität in einfacher Weise durch einen Vergleich der 1- oder 2-dimensionalen Proteinauftrennungen zwischen Wildtyp und gentechnisch veränderter Zelle bestimmt werden. Eine gebräuchliche Methode zur Präparation der Proteingele bei coryneformen Bakterien und zur Identifizierung der Proteine ist die von Hermann et al. (Electrophoresis, 22: 1712.23 (2001) beschriebene Vorgehensweise. Die Proteinkonzentration kann ebenfalls durch Western-Blot-Hybridisierung mit einem für das nachzuweisende Protein spezifischen Antikörper (Sambrook et al., Molecular Cloning: a laboratory manual, 2nd Ed. Cold Spring Harbor Laboratory Press, Cold Spring Harbor, N.Y. USA, 1989) und anschließender optische Auswertung mit entsprechender Software zur Konzentrationsbestimmung (Lohaus und Meyer (1989) Biospektrum, 5: 32-39; Lottspeich (1999), Angewandte Chemie 111: 2630-2647) analysiert werden. Die Aktivität von DNA-bindenden Proteinen kann mittels DNA-Band-Shift-Assays (auch als Gelretardation bezeichnet) gemessen werden (Wilson et al. (2001) Journal of Bacteriology, 183: 2151-2155). Die Wirkung von DNA-bindenden Proteinen auf die Expression anderer Gene kann durch verschiedene gut beschriebene Methoden des Reportergen-Assays nachgewiesen werden (Sambrook et al., Molecular Cloning: a laboratory manual, 2nd Ed. Cold Spring Harbor Laboratory Press, Cold Spring Harbor, N.Y. USA, 1989). Die intrazellulären enzymatischen Aktivitäten können nach verschiedenen beschriebenen Methoden (Donahue et al. (2000) Journal of Bacteriology 182 (19): 5624-5627; Ray et al. (2000) Journal of Bacteriology 182 (8): 2277-2284; Freedberg et al. (1973) Journal of Bacteriology 115 (3): 816-823) bestimmt werden. Sofern in den nachfolgenden Ausführungen keine konkreten Methoden zur Bestimmung der Aktivität eines bestimmten Enzyms angegeben werden, erfolgt die Bestimmung der Steigerung der Enzymaktivität und auch die Bestimmung der Verminderung einer Enzymaktivität vorzugsweise mittels der in Hermann et al., Electophoresis, 22: 1712-23 (2001), Lohaus et al., Biospektrum 5 32-39 (1998), Lottspeich, Angewandte Chemie 111: 2630-2647 (1999) und Wilson et al., Journal of Bacteriology 183: 2151-2155 (2001) beschriebenen Methoden.

Wird die Erhöhung der Enzymaktivität durch Mutation des endogenen Gens bewerkstelligt, so können derartige Mutationen entweder nach klassischen Methoden ungerichtet erzeugt werden, wie etwa durch UV-Bestrahlung oder durch mutationsauslösende Chemikalien, oder gezielt mittels gentechnologischer Methoden wie Deletion(en), Insertion(en) und/oder Nukleotidaustausch(e). Durch diese Mutationen werden veränderte Zellen erhalten. Besonders bevorzugte Mutanten von Enzymen sind insbesondere auch solche Enzyme, die nicht mehr oder zumindest im Vergleich zum Wildtyp-Enzym vermindert feedback-inhibierbar sind.

Wird die Erhöhung der Enzymaktivität durch Erhöhung der Synthese eines Enzyms bewerkstelligt, so erhöht man beispielsweise die Kopienzahl der entsprechenden Gene oder mutiert die Promotor- und Regulationsregion oder die Ribosomenbindungsstelle, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich, die Expression zu jedem beliebigen Zeitpunkt zu steigern. Des Weiteren können dem Enzym-Gen als regulatorische Sequenzen aber auch sogenannte "Enhancer" zugeordnet sein, die über eine verbesserte Wechselwirkung zwischen RNA-Polymerase und DNA ebenfalls eine erhöhte Genexpression bewirken. Durch Maßnahmen zur Verlängerung der Lebensdauer der mRNA wird ebenfalls die Expression verbessert. Weiterhin wird durch Verhinderung des Abbaus des Enzymproteins ebenfalls die Enzymaktivität verstärkt. Die Gene oder Genkonstrukte liegen dabei entweder in Plasmiden mit unterschiedlicher Kopienzahl vor oder sind im Chromosom integriert und amplifiziert. Alternativ kann weiterhin eine Überexpression der betreffenden Gene durch Veränderung der Medienzusammensetzung und Kulturführung erreicht werden. Anleitungen hierzu findet der Fachmann unter anderem bei Martin et al. (Bio/Technology 5, 137-146 (1987)), bei Guerrero et al. (Gene 138, 35-41 (1994)), Tsuchiya und Morinaga (Bio/Technology 6, 428-430 (1988)), bei Eikmanns et al. (Gene 102, 93-98 (1991)), in EP-A-0 472 869, im US 4,601,893, bei Schwarzer und Pühler (Bio/Technology 9, 84-87 (1991), bei Reinscheid et al. (Applied and Environmental Microbiology 60, 126-132 (1994)), bei LaBarre et al. (Journal of Bacteriology 175, 1001-1007 (1993)), in WO-A-96/15246, bei Malumbres et al. (Gene 134, 15-24 (1993), in JP-A-10-229891, bei Jensen und Hammer (Biotechnology and Bioengineering 58, 191-195 (1998)) und in bekannten Lehrbüchern der Genetik und Molekularbiologie. Die vorstehend beschriebenen Maßnahmen führen ebenso wie die Mutationen zu gentechnisch veränderten Zellen.

Zur Erhöhung der Expression der jeweiligen Gene werden zum Beispiel episomale Plasmide eingesetzt. Als Plasmide bzw. Vektoren kommen im Prinzip alle dem Fachmann für diesen Zweck zur Verfügung stehenden Ausführungsformen in Frage. Derartige Plasmide und Vektoren können z. B. den Broschüren der Firmen Novagen, Promega, New England Biolabs, Clontech oder Gibco BRL entnommen werden. Weitere bevorzugte Plasmide und Vektoren können gefunden werden in: Glover, D. M. (1985), DNA cloning: a practical approach, Vol. I-III, IRL Press Ltd. , Oxford; Rodriguez, R.L. und Denhardt, D. T (eds) (1988), Vectors : a survey of molecular cloning vectors and their uses, 179-204, Butterworth, Stoneham; Goeddel, D. V. (1990), Systems for heterologous gene expression, Methods Enzymol. 185, 3-7; Sambrook, J.; Fritsch, E. F. und Maniatis, T. (1989), Molecular cloning: a laboratory manual, 2nd ed., Cold Spring Harbor Laboratory Press, New York.

Der Plasmidvektor, der das zu amplifizierende Gen enthält, wird anschließend durch Konjugation oder Transformation in den gewünschten Stamm überführt. Die Methode der Konjugation ist beispielsweise bei Schäfer et al., Applied and Environmental Microbiology 60: 756-759 (1994) beschrieben. Methoden zur Transformation sind beispielsweise bei Thierbach et al., Applied Microbiology and Biotechnology 29: 356-362 (1988), Dunican und Shivnan, Bio/Technology 7: 1067-1070 (1989) und Tauch et al., FEMS Microbiology Let-ters 123: 343-347 (1994) beschrieben. Nach homologer Rekombination mittels eines "cross-over"-Ereignisses enthält der resultierende Stamm mindestens zwei Kopien des betreffenden Gens.

Unter der vorstehend und in den nachfolgenden Ausführungen verwendeten Formulierung "eine im Vergleich zu ihrem Wildtyp gesteigerte Aktivität eines Enzyms Ex" ist vorzugsweise stets eine um einen Faktor von mindestens 2, besonders bevorzugt von mindestens 10, darüber hinaus bevorzugt von mindestens 100, darüber hinaus noch mehr bevorzugt von mindestens 1.000 und am meisten bevorzugt von mindestens 10.000 gesteigerte Aktivität des jeweiligen Enzyms Ex zu verstehen. Weiterhin umfasst die erfindungsgemäße Zelle, welche "eine gegenüber ihrem Wildtyp gesteigerte Aktivität eines Enzyms Eₓ" aufweist, insbesondere auch eine Zelle, deren Wildtyp keine oder zumindest keine nachweisbare Aktivität dieses Enzyms Eₓ aufweist und die erst nach Erhöhung der Enzymaktivität, beispielsweise durch Überexpression, eine nachweisbare Aktivität dieses Enzyms Eₓ zeigt. In diesem Zusammenhang umfasst der Begriff "Überexpression" oder die in den nachfolgenden Ausführungen verwendete Formulierung "Erhöhung der Expression" auch den Fall, dass eine Ausgangszelle, beispielsweise eine Wildtyp-Zelle, keine oder zumindest keine nachweisbare Expression aufweist und erst durch rekombinante Verfahren eine nachweisbare Synthese des Enzyms Eₓ induziert wird.

Unter der nachfolgend verwendeten Formulierung "verminderte Aktivität eines Enzyms Eₓ" wird dementsprechend vorzugsweise eine um einen Faktor von mindestens 0,5, besonders bevorzugt von mindestens 0,1, darüber hinaus bevorzugt von mindestens 0,01, darüber hinaus noch mehr bevorzugt von mindestens 0,001 und am meisten bevorzugt von mindestens 0,0001 verminderte Aktivität verstanden. Die Formulierung "verminderte Aktivität" beinhaltet auch keine detektierbare Aktivität ("Aktivität von null"). Die Verminderung der Aktivität eines bestimmten Enzyms kann beispielsweise durch gezielte Mutation, durch Zugabe von kompetitiven oder nicht kompetitiven Inhibitoren oder durch andere, dem Fachmann bekannte Maßnahmen zur Verminderung der Aktivität eines bestimmten Enzyms erfolgen.

Es ist bevorzugt, dass die erfindungsgemäße Bakterienzelle, die im Vergleich zu ihrem Wildtyp mehr 2-Hydroxyisobuttersäure über Acetoacetyl-Coenzym A als Zwischenprodukt und 3-Hydroxybutyryl-Coenzym A als Vorprodukt zu bilden vermag, wobei die Zelle mindestens eine Aktivität eines Enzyms E₃ aufweist, welches die Umsetzung von 3-Hydroxybutyryl-Coenzym A zu 2-Hydroxyisobutyryl-Coenzym A katalysiert,
wobei die Zelle eine im Vergleich zu ihrem Wildtyp erhöhte Aktivität des Enzyms E₃ aufweist,
wobei die Zelle als Wildtyp eine Aktivität eines Enzyms E₄ aufweist, welches die Umsetung von 3-HydroxybutyrylCoenzym A zu Polyhydroxybutyrat katalysiert
wobei die Zelle eine im Vergleich zu ihrem Wildtyp erniedrigte Aktivität des Enzyms E₄ aufweist und wobei das Enzym E₃ ausgewählt wird aus einer HydroxylIsobutyryl-CoA Mutase, einer Isobutyryl-CoA Mutase (EC 5.4.99.13) oder einer Methylmalonyl-CoA Mutase (EC 5.4.99.2), und
wobei das Enzym E₄ eine Polyhydroxyalkanoat Synthase ist Kohlenhydrate, Glycerin, Öle und Fette, Kohlendioxid, Carbonsäuren oder Methanol als Kohlenstoffquelle zu verwerten vermag.

Weiterhin ist es für die erfindungsgemäße Bakterienzelle, die 2-Hydroxyisobuttersäure über Acetoacetyl-Coenzym A als Zwischenprodukt und 3-Hydroxybutyryl-Coenzym A als Vorprodukt zu bilden vermag, wobei die Zelle mindestens eine Aktivität eines Enzyms E₃ aufweist, welches die Umsetzung von 3-Hydroxybutyryl-Coenzym A zu 2-Hydroxyisobutyryl-Coenzym A katalysiert,
wobei die Zelle eine im Vergleich zu ihrem Wildtyp erhöhte Aktivität des Enzyms E₃ aufweist,
wobei die Zelle als Wildtyp eine Aktivität eines Enzyms E₄ aufweist, welches die Umsetung von 3-HydroxybutyrylCoenzym A zu Polyhydroxybutyrat katalysiert
wobei die Zelle eine im Vergleich zu ihrem Wildtyp erniedrigte Aktivität des Enzyms E₄ aufweist und wobei das Enzym E₃ ausgewählt wird aus einer HydroxylIsobutyryl-CoA Mutase, einer Isobutyryl-CoA Mutase (EC 5.4.99.13) oder einer Methylmalonyl-CoA Mutase (EC 5.4.99.2), und
wobei das Enzym E₄ eine Polyhydroxyalkanoat Synthase ist bevorzugt, dass die Zelle, gegebenenfalls zusätzlich zur Aktivität des Enzyms E₁, eine Aktivität eines Enzyms E₂, bevorzugt eine im Vergleich zu ihrem Wildtyp gesteigerte Aktivität des Enzyms E₂, aufweist, welche die Umsetzung von zwei Acetyl-Coenzym A zu Acetoacetyl-Coenzym A katalysiert. Bei dem Enzym E₂ handelt es sich vorzugsweise um eine Acetyl-CoA C-Acetyltransferase (EC-Number 2.3.1.9). Dieses Enzym wird vorzugsweise von den Genen kodiert ausgewählt aus der Gruppe bestehend aus acat1, acat2, loc484063, loc489421, mgc69098, mgc81403, mgc81256, mgc83664, kat-1, erg10, ygeF, atoB, fadAx, phbA-1, phbA-2, atoB-2, pcaF, pcaF-2, phb-A, bktB, phaA, tioL, thlA, fadA, paaJ, phbAf, pimB, mmgA, yhfS, thl, vraB, th1, mvaC, thiL, paaJ, fadA3, fadA4, fadA5, fadA6, cgl12392, catF, sc8f4.03, thiL1, thiL2, acaB1, acaB2, acaB3 oder acaB4, wobei a-cat1, acat2, atoB, *thlA, thlB,* phaA und phbA, besonders bevorzugt sind phaA und phbA.

Die Nukleotidsequenz dieser Gene können beispielsweise der "Kyoto Encyclopedia of Genes and Genomes" (KEGG-Datenbank), den Datenbanken des National Center for Biotechnology Information (NCBI) der National Library of Medicine (Bethesda, MD, USA) oder der Nukleotidsequenz-Datenbank der European Molecular Biologies Laboratories (EMBL, Heidelberg, Deutschland bzw. Cambridge, UK) entnommen werden.

Bei dem Enzym E₃ handelt es sich bevorzugt um solche Enzyme, die sich aus den Mikroorganismen Aquincola tertiaricarbonis L108, DSM18028, DSM18512, Methylibium petroleiphilum PM1, Methylibium sp. R8, Xanthobacter autotrophicus Py2, Rhodobacter sphaeroides (ATCC 17029), Nocardioides sp. JS614, Marinobacter algicola DG893, Sinorhizobium medicae WSM419, Roseovarius sp. 217, Pyrococcus furiosus DSM 3638 isolieren lassen, besonders bevorzugt um die in WO 2007/110394 beschriebenen Coenzym B12-abhängigen Mutase, sowie Enzyme, die in mindestens einer ihrer Teilsequenz eine Sequenzidentität zu der Aminosäuresequenz der kleinen bzw. großen Untereinheit der in WO 2007/110394 beschriebenen Mutase (accession number DQ436457.1 und DQ436456.1) von mindestens 60%, vorzugsweise von mindestens 80%, besonders bevorzugt von mindestens 95%, ganz besonders bevorzugt mindestens 99% auf Aminosäureebene aufweisen, bestimmt nach dem blastp Algorithmus mit einem expect threshold von 10, einer word size von 3, einer blosum62 Matrix mit gap costs von existence:
11 und extension: 1 und einem conditional compositional score matrix adjustment.

Bei dem Enzym E₄ handelt es sich vorzugsweise um eine Polyhydroxyalkanoat Synthase, besonders bevorzugt um eine Polyhydroxybutyrat Synthase. Dieses Enzym wird vorzugsweise von den Genen phbC oder phaC kodiert, wobei phaC besonders bevorzugt ist.

In einer weiteren, bevorzugten Ausführungsform der erfindungsgemäßen Bakterienzelle, die 2-Hydroxyisobuttersäure über Acetoacetyl-Coenzym A als Zwischenprodukt und 3-Hydroxybutyryl-Coenzym A als Vorprodukt zu bilden vermag, wobei die Zelle mindestens eine Aktivität eines Enzyms E₃ aufweist, welches die Umsetzung von 3-Hydroxybutyryl-Coenzym A zu 2-Hydroxyisobutyryl-Coenzym A katalysiert,
wobei die Zelle eine im Vergleich zu ihrem Wildtyp erhöhte Aktivität des Enzyms E₃ aufweist,
wobei die Zelle als Wildtyp eine Aktivität eines Enzyms E₄ aufweist, welches die Umsetung von 3-HydroxybutyrylCoenzym A zu Polyhydroxybutyrat katalysiert
wobei die Zelle eine im Vergleich zu ihrem Wildtyp erniedrigte Aktivität des Enzyms E₄ aufweist und wobei das Enzym E₃ ausgewählt wird aus einer HydroxylIsobutyryl-CoA Mutase, einer Isobutyryl-CoA Mutase (EC 5.4.99.13) oder einer Methylmalonyl-CoA Mutase (EC 5.4.99.2), und
wobei das Enzym E₄ eine Polyhydroxyalkanoat Synthase ist, weist die erfindungsgemäße Zelle als
Wildtyp eine Aktivität eines Enzyms E₅, bevorzugt eine im Vergleich zu ihrem Wildtyp erniedrigte Aktivität eines Enzyms E₅, auf, welches die Umsetzung von 3-Hydroxybutyryl-Coenzym A zu Crotonyl-Coenzym A katalysiert.

Bei dem Enzym E₅ handelt es sich vorzugsweise um eine Crotonase (EC-Number 4.2.1.55) oder um eine (3R)-3-Hydroxybutanoyl-CoA Dehydratase (EC-Number 4.2.1.17). Dieses Enzym wird vorzugsweise von den Genen kodiert ausgewählt aus der Gruppe bestehend aus crt, crt1, crt2, fadB, paaF, wobei crt sowie das entsprechende Gen aus Clostridien bevorzugt, *crt* aus *Clostridium acetobutylicum* besonders bevorzugt sind.

In noch einer weiteren, bevorzugten Ausführungsform der erfindungsgemäßen Bakterienzelle, die 2-Hydroxyisobuttersäure über Acetoacetyl-Coenzym A als Zwischenprodukt und 3-Hydroxybutyryl-Coenzym A als Vorprodukt zu bilden vermag, wobei die Zelle mindestens eine Aktivität eines Enzyms E₃ aufweist, welches die Umsetzung von 3-Hydroxybutyryl-Coenzym A zu 2-Hydroxyisobutyryl-Coenzym A katalysiert,
wobei die Zelle eine im Vergleich zu ihrem Wildtyp erhöhte Aktivität des Enzyms E₃ aufweist,
wobei die Zelle als Wildtyp eine Aktivität eines Enzyms E₄ aufweist, welches die Umsetung von 3-HydroxybutyrylCoenzym A zu Polyhydroxybutyrat katalysiert
wobei die Zelle eine im Vergleich zu ihrem Wildtyp erniedrigte Aktivität des Enzyms E₄ aufweist und wobei das Enzym E₃ ausgewählt wird aus einer HydroxylIsobutyryl-CoA Mutase, einer Isobutyryl-CoA Mutase (EC 5.4.99.13) oder einer Methylmalonyl-CoA Mutase (EC 5.4.99.2), und
wobei das Enzym E₄ eine Polyhydroxyalkanoat Synthase ist, weist die erfindungsgemäße Zelle eine Aktivität eines Enzyms E₆, bevorzugt eine im Vergleich zu ihrem Wildtyp erniedrigte Aktivität eines Enzyms E₆, auf, welches die Umsetzung von R-3-Hydroxybutyryl-Coenzym A zu S-3-Hydroxybutyryl-Coenzym A katalysiert.

Bei dem Enzym E₆ handelt es sich vorzugsweise um eine 3-Hydroxybutyryl-CoA Epimerase (EC 5.1.2.3). Dieses Enzym wird vorzugsweise von den Genen kodiert ausgewählt aus der Gruppe bestehend aus fadB, fadB1, fadB2, fadJ, fabJ-1, faoA yfcX wobei fadB, fadJ, yfcX bevorzugt, fadB, fadJ besonders bevorzugt sind.

Weiterhin ist es für die erfindungsgemäße Bakterienzelle, die 2-Hydroxyisobuttersäure über Acetoacetyl-Coenzym A als Zwischenprodukt und 3-Hydroxybutyryl-Coenzym A als Vorprodukt zu bilden vermag, wobei die Zelle mindestens eine Aktivität eines Enzyms E₃ aufweist, welches die Umsetzung von 3-Hydroxybutyryl-Coenzym A zu 2-Hydroxyisobutyryl-Coenzym A katalysiert,
wobei die Zelle eine im Vergleich zu ihrem Wildtyp erhöhte Aktivität des Enzyms E₃ aufweist,
wobei die Zelle als Wildtyp eine Aktivität eines Enzyms E₄ aufweist, welches die Umsetung von 3-HydroxybutyrylCoenzym A zu Polyhydroxybutyrat katalysiert
wobei die Zelle eine im Vergleich zu ihrem Wildtyp erniedrigte Aktivität des Enzyms E₄ aufweist und wobei das Enzym E₃ ausgewählt wird aus einer HydroxylIsobutyryl-CoA Mutase, einer Isobutyryl-CoA Mutase (EC 5.4.99.13) oder einer Methylmalonyl-CoA Mutase (EC 5.4.99.2), und
wobei das Enzym E₄ eine Polyhydroxyalkanoat Synthase ist, bevorzugt, dass die erfindungsgemäße Zelle eine im Vergleich zu ihrem Wildtyp erniedrigte Aktivität mindestens eines Enzyms E₇ aufweist, welches 3-Hydroxybutyryl-Coenzym A als Substrat akzeptiert.

Einen weiteren Beitrag zur Lösung der eingangs genannten Aufgaben leistet ein Verfahren zur Herstellung von 2-Hydroxyisobuttersäure oder von 2-Hydroxyisobuttersäure-Monomereinheiten enthaltende Polyhydroxyalkanoaten, umfassend die Verfahrensschritte:
a) in Kontakt Bringen einer erfindungsgemäßen Bakterienzelle mit einem Nährmedium beinhaltend eine Kohlenstoffquelle unter Bedingungen, unter denen aus der Kohlenstoffquelle 2-Hydroxyisobuttersäure oder 2-Hydroxyisobuttersäure-Monomereinheiten enthaltende Polyhydroxyalkanoate gebildet werden, sowie gegebenenfalls
b) Aufreinigung der 2-Hydroxyisobuttersäure aus dem Nährmedium oder 2-Hydroxyisobuttersäure-Monomereinheiten enthaltende Polyhydroxyalkanoate aus den Zellen.

Als Kohlenstoffquelle können beispielsweise Kohlenhydrate [wie z. B. Monosaccharide (z.B. Glucose, Fructose, Galactose, Arabinose, Xylose), Oligosaccharide (z. B. Maltose, Saccharose, Lactose), und Polysacharide (z.B. Stärke, hydrolytisch behandelte Stärke, Cellulose , hydrolytisch behandelte Cellulose, Hemicellulose, hydrolytisch behandelte Hemicellulose)], sowie deren Reaktionsprodukte, wie z.B. Zuckeralkohole und Polyhydroxysäuren; Kohlendioxid;
organische, gegebenenfalls 1 oder mehrere, z. B. 1 , 2, 3 oder 4 Hydroxylgruppen tragende Mono-, Di- und Tricarbonsäuren, z. B. Essigsäure, Weinsäure, Itaconsäure, Bernsteinsäure, Propionsäure, Milchsäure, 3-Hydroxypropionsäure, Fumarsäure, Maleinsäure, 2,5-Furandicarbonsäure, Glutarsäure, Lävulinsäure, Gluconsäure, Aconitsäure, Bernsteinsäure und Diaminopimelinsäure, Zitronensäure;
Lipide;
Öle oder Fette wie z. B. Rapsöl, Sojaöl, Palmöl, Sonnenblumenöl, Erdnussöl und Kokosfett;
gesättigte und ungesättigte Fettsäuren mit vorzugsweise 10 bis 22 C-Atomen, z. B. γ-Linolensäure, Dihomo-γ-Linolensäure, Arachidonsäure, Palmitinsäure, Stearinsäure, Linolsäure, Eicosapentaensäure und Docosahexaensäure;
Kohlenwasserstoffe wie Methan;
Alkohole, z. B. mit 1 bis 22 C-Atomen, z. B. Butanol, Methanol, Ethanol;
Diole mit vorzugsweise 3 bis 8 C-Atomen, z. B. Propandiol und Butandiol;
mehrwertige (auch bezeichnet als höherwertige) Alkohole mit 3 oder mehr, z. B. 3, 4, 5 oder 6 OH-Gruppen, z. B. Glycerin, Sorbitol, Manitol, Xylitol und Arabinitol;
Ketone mit vorzugsweise 3 bis 10 C-Atomen und gegebenenfalls 1 oder mehreren Hydroxylgruppen, z. B. Aceton und Acetoin; Lactone, z. B. γ-Butyrolacton, Cyclodextrine, Biopolymere, z. B. Polyhydroxyacetat, Polyester, z. B. Polylactid, Polysaccharide, Polyisoprenoide, Polyamide;
aromatische Verbindungen, z. B. aromatische Amine, Vanillin und Indigo;
Proteine, z. B. Enzyme wie Amylasen, Pektinasen, saure, hybride oder neutrale Zellulasen, Esterasen wie Lipasen, Pankreasen, Proteasen, Xylanasen und Oxidoreduktasen wie Laccase, Katalase und Peroxidase, Glucanasen, Phytasen; Carotenoide, z. B. Lycopin, β-Carotin, Astaxanthin, Zeaxanthin und Canthaxanthin; proteinogene und nicht-proteinogene Aminosäuren, z. B. Lysin, Glutamat, Methionin, Phenylalanin, Asparaginsäure, Tryptophan und Threonin;
Purin- und Pyrimidinbasen;
Nukleoside und Nukleotide, z. B. Nikotinamidadenindinukleotid (NAD) und Adenosin-5'-monophosphat (AMP);
sowie Vorstufen und Derivate, z.B. bei den genannten Säuren deren Salze, der vorgenannten Verbindungen
eingesetzt werden.

Diese Stoffe können einzeln oder als Mischung verwendet werden. Besonders bevorzugt ist der Einsatz von Kohlenhydraten, insbesondere Monosacchariden, Oligosacchariden oder Polysacchariden, wie dies beispielsweise in US 6,136,576 beschrieben ist, von C₅-Zuckern oder von Glycerin.

Methanol ist ein bevorzugt einzusetzender Alkohol, da es aus vielen verschiedenen Quellen wie beispielsweise Biogas, Biomasse, Erdgas oder Kohle hergestellt werden kann.

Die Kohlenstoffquellen können in unterschiedlicher Form (rein oder in Lösung/Suspension) und in unterschiedlichen Zusammensetzungen (aufgereinigt oder als Rohprodukt) aus unterschiedlichen Verarbeitungsstufen (z.B. Zuckerrohrsaft, Syrup, Melasse, Rohzucker, Kristallzucker; Maiskorn, Mehl, Stärke, Dextrin, Glucose), vor oder nach Behandlung (Dampfexplosion, Säurevorbehandlung, enzymatische Vorbehandlung) eingesetzt werden.

In einer alternativen Ausführungsform umfasst die Kohlenstoffquelle CO₂ oder CO, insbesondere Synthesegas. Die in diesem Zusammenhang eingesetzten erfindungsgemäßen Zellen sind acetogene Zellen, wie beispielsweise Arten der Gattung *Acetobacterium* wie *A. woodii* und *Clostridium aceticum.* Insbesondere sind die acetogenen Zellen ausgewählt aus der Gruppe umfassend *Thermoanaerobacter kivui, Acetobacterium woodii, Acetoanaerobium notera, Clostridium aceticum, Butyribacterium methylotrophicum, Clostridium acetobutylicum, Moorella thermoacetica, Eubacterium limosum, Peptostreptococcus productus, Clostridium ljungdahlii* und *Clostridium carboxidivorans.* Eine besonders geeignete Zelle ist in diesem Zusammenhang *Clostridium carboxidivorans,* insbesondere solche Stämme wie "P7" und "P11". Solche Zellen sind beispielsweise in US 2007/0275447 und US 2008/0057554 beschrieben. Eine weitere, in diesem Zusammenhang besonders geeignete Zelle ist *Clostridium ljungdahlii,* insbesondere Stämme ausgewählt aus der Gruppe umfassend *Clostridium ljungdahlii* PETC, *Clostridium ljungdahlii* ERI2, *Clostridium ljungdahlii* C0l und *Clostridium ljungdahlii* O-52 und werden in der WO 98/00558 und WO 00/68407 beschrieben.

Die erfindungsgemäßen, gentechnisch veränderten Bakterienzellen können kontinuierlich oder diskontinuierlich im batch-Verfahren (Satzkultivierung) oder im *fed-batch*-Verfahren (Zulaufverfahren) oder *repeated-fed-batch-Verfahren* (repetitives Zulaufverfahren) zum Zwecke der Produktion von 2-Hydroxyisobutyrat oder von 2-Hydroxyisobuttersäure-Monomereinheiten enthaltenden Polyhydroxyalkanoaten mit dem Nährmedium in Kontakt und somit kultiviert werden. Denkbar ist auch ein semi-kontinuierliches Verfahren, wie es beispielsweise in der GB-A-1009370 beschrieben wird. Eine Zusammenfassung über weitere bekannte Kultivierungsmethoden sind im Lehrbuch von Chmiel ("Bioprozesstechnik 1. Einführung in die Bioverfahrenstechnik" (Gustav Fischer Verlag, Stuttgart, 1991)) oder im Lehrbuch von Storhas ("Bioreaktoren und periphere Einrichtungen", Vieweg Verlag, Braunschweig/Wiesbaden, 1994) beschrieben. Das zu verwendende Kulturmedium muss in geeigneter Weise den Ansprüchen der jeweiligen Stämme genügen. Beschreibungen von Kulturmedien verschiedener Mikroorganismen sind im Handbuch "Manual of Methods for General Bacteriology" der American Society for Bacteriology (Washington D. C., USA, 1981) enthalten.

Als Stickstoffquelle können organische stickstoffhaltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammoniumnitrat verwendet werden. Die Stickstoffquellen können einzeln oder als Mischung verwendet werden.

Als Phosphorquelle können Phosphorsäure, Kaliumdihydrogenphosphat oder Dikaliumhydrogenphosphat oder die entsprechenden Natrium-haltigen Salze verwendet werden. Das Kulturmedium muss weiterhin Salze von Metallen enthalten wie z. B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können überdies geeignete Vorstufen zugesetzt werden. Die genannten Einsatzstoffe können zur Kultur in Form eines einmaligen Ansatzes hinzugegeben oder in geeigneter Weise während der Kultivierung zugefüttert werden.

Zur pH-Kontrolle der Kultur werden basische Verbindungen wie Natriumhydroxid, Kaliumhydroxid, Ammoniak bzw. Ammoniakwasser oder saure Verbindungen wie Phosphorsäure oder Schwefelsäure in geeigneter Weise eingesetzt. Zur Kontrolle der Schaumentwicklung können Antischaummittel wie z. B. Fettsäurepolyglykolester eingesetzt werden. Zur Aufrechterhaltung der Stabilität von Plasmiden können dem Medium geeignete selektiv wirkende Stoffe wie z. B. Antibiotika hinzugefügt werden. Um aerobe Bedingungen aufrechtzuerhalten, werden Sauerstoff oder Sauerstoff-haltige Gasmischungen, wie z. B. Luft, in die Kultur eingetragen. Die Temperatur der Kultur liegt normalerweise bei 20°C bis 45°C und vorzugsweise bei 25°C bis 40°C. Insbesondere bei der Verwendung von Zellen, welche Glycerin als Substrat umwandeln können, kann es bevorzugt sein, als Zellen solche Zellen einzusetzen, die in der US 6,803,218 beschrieben werden. In diesem Fall können die Zellen bei Temperaturen im Bereich von 40 bis 100°C kultiviert werden.

Die Aufreinigung der 2-Hydroxyisobuttersäure aus der Nährlösung erfolgt vorzugsweise kontinuierlich, wobei es in diesem Zusammenhang weiterhin bevorzugt ist, auch die Herstellung der 2-Hydroxyisobuttersäure durch Fermentation kontinuierlich durchzuführen, so dass der gesamte Prozess von der Herstellung der 2-Hydroxyisobuttersäure bis zur deren Aufreinigung aus der Fermentationsbrühe kontinuierlich durchgeführt werden kann. Zur kontinuierlichen Aufreinigung der Herstellung der 2-Hydroxyisobuttersäure aus der Fermentationsbrühe wird diese kontinuierlich über eine Vorrichtung zur Abtrennung der bei der Fermentation eingesetzten Mikroorganismen, vorzugsweise über einen Filter mit einer Ausschlußgröße in einem Bereich von 20 bis 200 kDa geführt, in dem eine Fest/Flüssig-Trennung stattfindet. Denkbar ist auch der Einsatz einer Zentrifuge, einer geeigneten Sedimentationsvorrichtung oder eine Kombination dieser Vorrichtungen, wobei es besonders bevorzugt ist, zumindest einen Teil der Mikroorganismen zunächst durch Sedimentation abzutrennen und anschließend die von den Mikroorganismen teilweise befreite Fermentationsbrühe einer Ultrafiltration oder Zentrifugationsvorrichtung zuzuführen.

Das hinsichtlich seines 2-Hydroxyisobuttersäure-Anteils angereicherte Fermentationserzeugnis wird nach der Abtrennung der Mikroorganismen einer vorzugsweise mehrstufigen Trennanlage zugeführt. In dieser Trennanlage sind mehrere hintereinander geschaltete Trennstufen vorgesehen, aus denen jeweils Rückführleitungen ausmünden, die zum Fermentationstank zurückgeführt sind. Weiterhin führen aus den jeweiligen Trennstufen Ableitungen heraus. Die einzelnen Trennstufen können nach dem Prinzip der Elektrodialyse, der Umkehrosmose, der Ultrafiltration oder der Nanofiltration arbeiten. In der Regel handelt es sich um Membran-Trenneinrichtungen in den einzelnen Trennstufen. Die Auswahl der einzelnen Trennstufen ergibt sich aus Art und Umfang der Gärungsnebenprodukte und Substratreste.

Neben der Abtrennung der 2-Hydroxyisobuttersäure mittels Elektrodialyse, Umkehrosmose, Ultrafiltration oder Nanofiltration, in deren Verlauf als Endprodukt eine wässrige 2-Hydroxyisobuttersäure-Lösung erhalten wird, kann die 2-Hydroxyisobuttersäure auch durch Extraktionsverfahren aus der von Mikroorganismen befreiten Fermentationslösung abgetrennt werden, wobei in diesem Fall letztendlich die reine 2-Hydroxyisobuttersäure erhalten werden kann. Zur Abtrennung der 2-Hydroxyisobuttersäure durch Extraktion können der Fermentationslösung beispielsweise Ammoniumverbindungen oder Amine zugesetzt werden, um ein Ammoniumsalz der 2-Hydroxyisobuttersäure zu bilden. Dieses Ammoniumsalz kann dann aus der Fermentationslösung abgetrennt werden, in dem ein organisches Extraktionsmittel zugesetzt und die so erhaltene Mischung anschließend erhitzt wird, wodurch das Ammoniumsalz sich in der organischen Phase anreichert. Aus dieser Phase kann die 2-Hydroxyisobuttersäure dann unter Erhalt der reinen 2-Hydroxyisobuttersäure beispielsweise durch weitere Extraktionsschritte isoliert werden. Genauere Einzelheiten bezüglich dieses Trennverfahrens sind der WO-A-02/090312 zu entnehmen. Je nach Art und Weise der Abtrennung der 2-Hydroxyisobuttersäure aus der Fermentationslösung wird entweder eine wässrige 2-Hydroxyisobuttersäure-Lösung, beinhaltend 2 bis 90 Gew.-%, vorzugsweise 7,5 bis 50 Gew.-% und besonders bevorzugt 10 bis 25 Gew.-% an 2-Hydroxyisobuttersäure, oder aber reine 2-Hydroxyisobuttersäure erhalten.

2-Hydroxyisobuttersäure tendiert mit steigenden Konzentrationen dazu, ihr cyclisches Dimer (Tetramethylglycolide, TMG) zu bilden. Dieses Dimer kann im Dehydratisierungsschritt des erfindungsgemäßen Verfahrens analog zur 2-Hydroxyisobuttersäure behandelt werden, und soll somit im Folgenden für diesen Verfahrenssschritt immer mit unter den Begriff "2-Hydroxyisobuttersäure" fallen.

Des Weiteren kann die durch das erfindungsgemäße Verfahren hergestellte 2-Hydroxyisobuttersäure vor, während oder nach der Aufreinigung noch neutralisiert werden, wobei hierzu beispielsweise Basen wie etwa Alkali- oder Erdalkali-Hydroxide, wie z.B. Kalziumhydroxid oder Natriumhydroxid oder auch beispielsweise NH₃ oder NH₄OH eingesetzt werden können.

Einen Beitrag zur Lösung der eingangs genannten Aufgaben leistet insbesondere auch ein Verfahren zur Herstellung von Methacrylsäure oder Methacrylsäureestern, umfassend die Verfahrensschritte:
IA) Herstellung von 2-Hydroxyisobuttersäure durch das vorstehend beschriebene Verfahren sowie gegebenenfalls Aufreinigung und/oder Neutralisation der 2-Hydroxyisobuttersäure,
IB) Dehydratisierung der 2-Hydroxyisobuttersäure unter Bildung von Methacrylsäure sowie gegebenenfalls Veresterung des Methacrylates bzw. der Methacrylsäure.

Gemäß Verfahrensschritt IB) wird die 2-Hydroxyisobuttersäure unter Bildung von Methacrylsäure dehydratisiert, wobei hierzu entweder die aus der Fermentationslösung isolierte, reine 2-Hydroxyisobuttersäure oder aber die bei der Aufarbeitung der Fermentationslösung isolierte wässrige 2-Hydroxyisobuttersäure-Lösung eingesetzt werden kann, wobei diese gegebenenfalls noch vor der Dehydratisierung beispielsweise durch Destillation, gegebenenfalls in Gegenwart eines geeigneten Schleppmittels, aufkonzentriert wird.

Die Dehydratisierung kann grundsätzlich in flüssiger Phase oder in der Gasphase durchgeführt werden. Weiterhin ist es erfindungsgemäß bevorzugt, dass die Dehydratisierung in Gegenwart eines Katalysators erfolgt, wobei die Art des eingesetzten Katalysators davon abhängig ist, ob eine Gasphasen- oder eine Flüssigphasenreaktion durchgeführt wird.

Als Dehydratisierungskatalysatoren kommen sowohl saure als auch alkalische Katalysatoren in Betracht. Saure Katalysatoren sind insbesondere wegen der geringen Neigung zur Oligomerenbildung bevorzugt. Der Dehydratisierungskatalysator kann sowohl als homogener als auch als heterogener Katalysator eingesetzt werden. Wenn der Dehydratisierungskatalysator als heterogener Katalysator vorliegt, ist es bevorzugt, dass der Dehydratisierungskatalysator mit einem Träger x in Kontakt steht. Als Träger x kommen alle dem Fachmann als geeignet erscheinende Feststoffe in Betracht. In diesem Zusammenhang ist es bevorzugt, dass diese Feststoffe geeignete Porenvolumina aufweisen, die zur guten Anbindung und Aufnahme des Dehydratisierungskatalysators geeignet sind. Außerdem sind Gesamtporenvolumen nach DIN 66133 in einem Bereich von 0,01 bis 3 ml/g bevorzugt und Gesamtporenvolumen in einem Bereich von 0,1 bis 1,5 ml/g besonders bevorzugt. Zudem ist es bevorzugt, dass die als Träger x geeigneten Feststoffe eine Oberfläche im Bereich von 0,001 bis 1000 m²/g, vorzugsweise im Bereich von 0,005 bis 450 m²/g und darüber hinaus bevorzugt im Bereich von 0,01 bis 300 m²/g nach BET-Test gemäß DIN 66131 aufweisen. Als Träger für den Dehydratisierungskatalysator kann zum einen ein Schüttgut, das einen mittleren Teilchendurchmesser im Bereich von 0,1 bis 40 mm, vorzugsweise im Bereich von 1 bis 10 mm und darüber hinaus bevorzugt im Bereich von 1,5 bis 5 mm aufweist, eingesetzt werden. Ferner kann die Wand des Dehydratisierungsreaktors als Träger dienen. Weiterhin kann der Träger an sich sauer oder basisch sein oder ein saurer oder basischer Dehydratisierungskatalysator auf einen inerten Träger aufgebracht werden. Als Aufbringtechniken sind insbesondere Eintauchen bzw. Imprägnieren oder das Einarbeiten in eine Trägermatrix zu nennen.

Als Träger x, die auch Dehydratisierungskatalysatoreigenschaften aufweisen können, eignen sich insbesondere natürliche oder synthetische silikatische Stoffe, wie insbesondere Mordenit, Montmorillonit, saure Zeolithe; mit ein-, zwei oder mehrbasigen anorganischen Säuren, insbesondere Phosphorsäure, oder saure Salze anorganischer Säuren belegte Trägerstoffe, wie oxidische oder silikatische Stoffe, beispielsweise Al₂O₃, TiO₂; Oxide und Mischoxide, wie beispielsweise γ-Al₂O₃ und ZnO-Al₂O₃-Mischoxide der Heteropolysäuren.

Es entspricht einer erfindungsgemäßen Ausführungsform, dass der Träger x mindestens teilweise aus einer oxidischen Verbindung besteht. Derartige oxidische Verbindungen sollten mindestens eines der Elemente aus Si, Ti, Zr, Al, P oder eine Kombination von mindestens zwei davon aufweisen. Derartige Träger können auch selber durch ihre sauren bzw. basischen Eigenschaften als Dehydratisierungskatalysator wirken. Eine bevorzugte sowohl als Träger als x als auch als Dehydratisierungskatalysator wirkende Verbindungsklasse beinhalten Silizium/Aluminium/Phosphoroxide. Bevorzugte basische sowohl als Dehydratisierungskatalysator als auch als Träger x fungierende Stoffe beinhalten Alkali, Erdalkali, Lanthan, Lanthanoide oder eine Kombination von mindestens zwei davon in ihrer oxidischen Form. Derartige saure oder basische Dehydratisierungskatalysatoren sind sowohl bei der Evonik Degussa GmbH als auch bei der Südchemie AG kommerziell erhältlich. Eine weitere Klasse stellen Ionenaustauscher dar. Auch diese können sowohl in basischer als auch in saurer Form vorliegen.

Als homogene Dehydratisierungskatalysatoren kommen insbesondere anorganische Säuren, vorzugsweise Phosphor beinhaltende Säuren und darüber hinaus bevorzugt Phosphorsäure in Betracht. Diese anorganischen Säuren können auf dem Träger x durch Eintauchen bzw. Imprägnieren immobilisiert werden.

Insbesondere bei der Gasphasendehydratisierung hat sich der Einsatz von heterogenen Katalysatoren besonders bewährt. Bei der Flüssigphasendehydratisierung werden jedoch sowohl homogene als auch heterogene Dehydratisierungskatalysatoren eingesetzt.

Zudem ist es bevorzugt, dass in dem erfindungsgemäßen Verfahren ein Dehydratisierungskatalysator mit einem H0-Wert im Bereich von +1 bis -10, vorzugsweise in einem Bereich von +2 bis -8,2 und darüber hinaus bevorzugt bei der Flüssigphasendehydratisierung in einem Bereich von +2 bis -3 und in der Gasphasendehydratisierung in einem Bereich von -3 bis - 8,2 eingesetzt wird. Der H0-Wert entspricht der Säurefunktion nach Hämmert und lässt sich durch die sogenannte Amin-Titration und Verwendung von Indikatoren oder durch Absorption einer gasförmigen Base ermitteln (siehe "Studies in Surface Science and Catalytics", Vol. 51, 1989: "New solid Acids and Bases, their catalytic Properties", K. Tannabe et al.

Gemäß einer besonderen Ausführungsform des erfindungsgemäßen Verfahrens wird als saurer Feststoffkatalysator ein mit einer anorganischen Säure, vorzugsweise mit Phosphorsäure oder mit Supersäuren wie etwa sulfatisiertem oder phosphatisiertem Zirkoniumoxid in Kontakt gebrachter porenförmiger Trägerkörper eingesetzt, der vorzugsweise zu mindestens 90 Gew.-%, darüber hinaus bevorzugt zu mindestens 95 Gew.-% und am meisten bevorzugt zu mindestens 99 Gew.-% auf einem Siliziumoxid, vorzugsweise auf SiO₂, basiert. Das in Kontakt bringen des porenförmigen Trägerkörpers mit der anorganischen Säure erfolgt vorzugsweise durch das Imprägnieren des Trägerkörpers mit der Säure, wobei diese vorzugsweise in einer Menge in einem Bereich von 10 bis 70 Gew.-%, besonders bevorzugt in einem Bereich 20 bis 60 Gew.-% und darüber hinaus bevorzugt in einem Bereich von 30 bis 50 Gew.-%, bezogen auf das Gewicht des Trägerkörpers, mit diesem in Kontakt gebracht und anschließend getrocknet wird. Im Anschluss an die Trocknung wird der Trägerkörper zur Fixierung der anorganischen Säure erhitzt, vorzugsweise auf eine Temperatur in einem Bereich von 300 bis 600 °C, darüber hinaus bevorzugt in einem Bereich von 400 bis 500 °C.

Gemäß einer besonderen Ausführungsform des erfindungsgemäßen Verfahrens wird die Dehydratisierung in der Gasphase durchgeführt. Dabei können übliche Apparaturen, wie sie dem Fachmann für Gasphasenreaktion bekannt sind, beispielsweise Röhrenreaktoren, eingesetzt werden. Besonders bevorzugt ist der Einsatz von Rohrbündelwärmeaustauschern sowie von Reaktoren, welche Thermobleche als Wärmeaustauscher beinhalten.

Gemäß einer Ausführungsform der Gasphasendehydratisierung wird reine 2-Hydroxyisobuttersäure in einen Reaktor umfassend einen der vorstehend genannten Festbettkatalysatoren eingebracht.

Gemäß einer anderen Ausführungsform wird die 2-Hydroxyisobuttersäure in Form einer wässrigen Lösung beinhaltend 2 bis 80 Gew.-%, besonders bevorzugt 5 bis 50 Gew.-% und darüber hinaus bevorzugt 10 bis 25 Gew.-% an 2-Hydroxyisobuttersäure, jeweils bezogen auf das Gesamtgewicht der wässrigen Lösung, in den Reaktor eingebracht. Die Druck- und Temperaturbedingungen im Inneren des Reaktors werden so gewählt, dass die 2-Hydroxyisobuttersäure bzw. die wässrigen Lösung bei ihrem Eintritt in den Reaktor in gasförmiger Form vorliegt. Die Dehydratisierung in der Gasphase erfolgt vorzugsweise im Temperaturbereich zwischen 200 und 400 °C, besonders bevorzugt zwischen 250 und 350 °C. Der Druck im Inneren des Reaktors liegt bei der Gasphasendehydratisierung vorzugsweise in einem Bereich von 0,1 bis 50 bar, besonders bevorzugt in einem Bereich von 0,2 bis 10 bar und am meisten bevorzugt in einem Bereich von 0,5 bis 5 bar.

Die Menge an in den Reaktor eingebrachter 2-Hydroxyisobuttersäure liegt bei der Gasphasendehydratisierung vorzugsweise in einem Bereich von 10 bis 100 Vol-%, besonders bevorzugt in einem Bereich von 20 bis 100 Vol-% und am meisten bevorzugt in einem Bereich von 30 bis 100 Vol-%.

Gemäß einer anderen besonderen Ausführungsform des erfindungsgemäßen Verfahrens wird die Dehydratisierung in der Flüssigphase durchgeführt. Die Flüssigphasendehydratisierung kann ebenfalls in allen dem Fachmann bekannten Apparaturen durchgeführt werden, in denen ein Fluid auf eine gewünschte Reaktionstemperatur erhitzt werden kann, wobei die Apparatur mit einem Druck beaufschlagt werden kann, der ausreicht, um die Reaktionskomponenten unter den gewünschten Temperaturbedingungen flüssig zu halten.

Gemäß einer besonderen Ausführungsform des erfindungsgemäßen Verfahrens umfasst das Verfahren der Flüssigphasendehydratisierung einen ersten Verfahrensschritt, bei dem reine 2-Hydroxyisobuttersäure oder eine wässrige Lösung beinhaltend 5 bis 100 Gew.-%, besonders bevorzugt 20 bis 100 Gew.-% und am meisten bevorzugt 50 bis 100 Gew.-% an 2-Hydroxyisobuttersäure, bezogen auf das Gesamtgewicht der wässrigen Lösung, in einen Reaktor eingebracht wird. Die Druck- und Temperaturbedingungen im Inneren des Reaktors werden so gewählt, dass die 2-Hydroxyisobuttersäure bzw. die wässrigen Lösung bei ihrem Eintritt in den Reaktor in flüssiger Form vorliegt. Gemäß einer besonderen Ausführungsform des erfindungsgemäßen Verfahrens, bei dem eine Dehydratisierung in der Flüssigphase durchgeführt wird, wird die 2-Hydroxyisobuttersäure bzw. die wässrige Lösung im Inneren des Dehydratisierungsreaktors derart über ein Katalysatorfestbett geführt, dass die flüssige Phase über die Oberfläche der Katalysatorpartikel rieselt. Eine derartige Vorgehensweise kann beispielsweise in einem Rieselbettreaktor durchgeführt werden.

Die Dehydratisierung in der Flüssigphase erfolgt vorzugsweise im Temperaturbereich zwischen 200 und 350°C, besonders bevorzugt zwischen 250 und 300°C. Der Druck im Inneren des Reaktors liegt bei der Flüssigphasendehydratisierung vorzugsweise in einem Bereich von 1 bis 50 bar, besonders bevorzugt in einem Bereich von 2 bis 25 bar und am meisten bevorzugt in einem Bereich von 3 bis 10 bar.

Die Katalyse der Dehydratisierung kann sowohl bei der Gasphasendehydratisierung als auch bei der Flüssigphasendehydratisierung homogen oder heterogen erfolgen.

Bei der homogenen Katalyse wird der Katalysator, bei dem es sich dann vorzugsweise um eine anorganische Säure wie etwa Phosphorsäure oder Schwefelsäure handelt, zunächst mit der reinen 2-Hydroxyisobuttersäure oder mit der wässrigen Lösung beinhaltend die 2-Hydroxyisobuttersäure in Kontakt gebracht. Anschließend wird die so erhaltene Zusammensetzung in den Reaktor eingebracht und unter den gewünschten Druck- und Temperaturbedingungen in Methacrylsäure überführt. Denkbar ist auch, die anorganische Säure unabhängig von der 2-Hydroxyisobuttersäure bzw. der wässrigen Lösung in den Reaktor einzubringen. In diesem Fall weist der Reaktor mindestens zwei Zuleitungen, eine für die 2-Hydroxyisobuttersäure bzw. die wässrige Lösung beinhaltend die 2-Hydroxyisobuttersäure und eine für den Katalysator, auf. Wird die Dehydratisierung in flüssiger Phase in einem Rieselbettreaktor durchgeführt, so ist es bevorzugt, dass der Katalysator zusammen mit der 2-Hydroxyisobuttersäure bzw. der wässrigen Lösung beinhaltend die 2-Hydroxyisobuttersäure im Kopfbereich des Reaktors eingebracht wird.

Bei der heterogenen Katalyse befindet sich der Katalysator in Form eines festen Substrates im Reaktionsraum, beispielsweise in Form einer Festbettschüttung, in Form von mit Katalysator beschichteten Platten, vorzugsweise Thermoblechen, welche im Inneren des Reaktors angeordnet sind oder aber in Form von mit Katalysator beschichteten Reaktorwänden. Mögliche Reaktoren sind beispielsweise in der DE-A-198 48 208, DE-A-100 19 381 und EP-A-I 234 612 beschrieben. Im Falle der heterogenen Katalyse sind als Katalysatoren mit anorganischen Säuren in Kontakt gebrachte, vorzugsweise imprägnierte porenförmiger Trägerkörper bevorzugt. Die 2-Hydroxyisobuttersäure bzw. die wässrige Lösung beinhaltend die 2-Hydroxyisobuttersäure wird dann in dampfförmiger oder flüssiger Form mit der Oberfläche des festen Katalysatormaterials in Kontakt gebracht.

Gemäß einer besonders bevorzugten Ausführungsform des erfindungsgemäßen Verfahrens wird die Dehydratisierung der 2-Hydroxyisobuttersäure in flüssiger Phase bei einem Druck in einem Bereich von 200 bis 500 mbar, bei einer Temperatur in einem Bereich von 160 bis 300°C, bevorzugt von 200 bis 240 °C und in Gegenwart von Alkalimetall-Ionen als Katalysator durchgeführt.

Die entstandene Methacrylsäure kann bei den vorliegenden Reaktionsbedingungen gasförmig zusammen mit Wasser abdestilliert und im Anschluss als wässrige Lösung kondensiert werden, so dass eine wässrige Methacrylsäure-Lösung, die keine Katalysatorbestandteile enthält, erhalten werden kann.

Die so erhaltene Methacrylsäure-Lösung kann gemäß einer besonderen Ausführungsform des erfindungsgemäßen Verfahrens ohne weitere Aufarbeitung gegebenenfalls der Veresterung zugeführt werden. Dabei wird die Methacrylsäure-Lösung mit entsprechenden Alkoholen sowie geeigneten, dem Fachmann bekannten Veresterungkatalysatoren, wie etwa konzentrierten Säuren, unter Erhitzen in Kontakt gebracht und die Methacrylsäure auf diese Weise in die entsprechenden Ester überführt.

Zu den bevorzugten Alkoholen gehören unter anderem Alkohole mit jeweils mindestens einem Kohlenstoffatom, bevorzugt 2 bis 12 und besonders bevorzugt 4 bis 9 Kohlenstoffatomen. Die Alkohole können eine lineare, verzweigte oder cyclische Struktur aufweisen. Des Weiteren können die Alkohole aromatische Gruppen oder Substituenten, beispielsweise Halogenatome umfassen. Zu den bevorzugten Alkoholen gehören insbesondere Methanol, Ethanol, n-Propanol, iso-Propanol, n-Butanol, 1-Methyl-propanol, 2-Methyl-propanol, tert.-Butanol, n-Pentanol, 1-Methyl-butanol, 2-Methyl-butanol, 3-Methyl-butanol, 2,2-Dimethyl-propanol, n-Hexanol, 1-Methyl-pentanol, 2-Methyl-pentanol, 3-Methyl- pentanol, 4-Methyl-pentanol, 1,1-Dimethyl-butanol, 2,2-Dimethyl-butanol, 3,3-Dimethyl- butanol, 1 ,2-Dimethyl-butanol, n-Heptanol, 1-Methyl-hexanol, 2-Methyl-hexanol, 3- Methyl-hexanol, 4-Methyl-hexanol, 1 ,2-Dimethyl-pentanol, 1, 3-Dimethyl-pentanol, 1,1-Dimethyl-pentanol, 1,1, 2, 2-Tetramethyl-propanol, Benzylalkohol, n-Octanol, 2- Ethylhexanol, n-Nonanol,1-Methyl-octanol, 2-Methyl-octanol, n-Decanol, n-Undecanol, 1-Methyl-decanol, 2-Methyl-decanol, n-Dodecanol, 2,4-Diethyl-octanol, Cyclopentanol, Cyclohexanol, 4-tert.-Butyl-cyclohexanol, Cycloheptanol, Cyclododecanol, 2-(Dimethylamino)-ethanol, 3-(Dimethylamino)-propanol, 4-(Dimethylamino)-butanol, 5-(Dimethylamino)-pentanol, 6-(Dimethylamino)-hexanol, 8-(Dimethylamino)-octanol, 10-(Dimethylamino)-decanol, 12-(Dimethylamino)-dodecanol, 2-(Diethylamino)-ethanol, 3-(Diethylamino)-propanol, 4-(Diethylamino)-butanol, 5 - (Diethylamino)-pentanol, 6-(Diethylamino)-hexanol, 8-(Diethylamino)-octanol, 10-(Diethylamino)-decanol, 12-(Diethylamino)-dodecanol, 2-(Di- (iso-propyl)-amino)-ethanol, 3-(Di-(iso-propyl)- amino)-propanol, 4-(Di-(iso- propyl)-amino)-butanol, 5-(Di-(iso-propyl)-amino)-pentanol, 6-Di-(iso-propyl)-amino)-hexanol, 8-(Di-(iso-propyl)-amino)-octanol, 10-(Di-(isopropyl)- amino)-decanol, 12-(Di-(iso-propyl)-amino)-dodecanol, 2-(Dibutylamino)-ethanol, 3 -(Dibutylamino)-propanol, 4-(Dibutylamino)-butanol, 5 -(Dibutylamino)-pentanol, 6-(Dibutylamino)-hexanol, 8-(Dibutylamino)-octanol, 10-(Dibutylamino)-decanol, 12-(Dibutylamino)-dodecanol, 2-(Dihexylamino)-ethanol, 3 -(Dihexylamino)-propanol, 4-(Dihexylamino)-butanol, 5 -(Dihexylamino)-pentanol, 6-(Dihexylamino)-hexanol, 8-(Dihexylamino)-octanol, 10-(Dihexylamino)-decanol, 12-(Dihexylamino)-dodecanol, 2-(Methyl-ethyl-amino)-ethyi-, 2-(Methyl-propyl-amino)-ethanol, 2-(Methyl-iso-propyl- amino)- ethanol, 2-(Methyl-butyl-amino)-ethanol, 2-(Methyl-hexyl-amino)-ethanol, 2-(Methyl-octyl-amino)-ethanol, 2-(Ethyl-propyl-amino)-ethanol, 2-(Ethyl-iso-propyl- amino)-ethanol, 2-(Ethyl-butyl-amino)-ethanol, 2-(Ethyl-hexyl-amino)-ethanol, 2-(Ethyl- octyl-amino)-ethanol, 3-(Methyl-ethyl-amino)-propanol, 3-(Methyl-propyl-amino)- propanol, 3-(Methyl-isopropyl-amino)-propanol, 3-(Methyl-butyl-amino)-propanol, 3-(Methyl-hexyl-amino)-propanol, 3-(Methyl-octyl-amino)-propanol, 3-(Ethyl-propyl- amino)-propanol, 3-(Ethyl-iso-propyl-amino)-propanol, 3-(Ethyl-butyl-amino)-propanol, 3-(Ethyl-hexyl-amino)-propanol, 3-(Ethyl-octyl-amino)-propanol, 4-(Methyl-ethyl-amino)- butanol, 4-(Methyl-propyl-amino)-butanol, 4-(Methyl-isopropyl-amino)-butanol, 4-(Methyl-butyl-amino)-butanol, 4-(Methyl-hexyl-amino)-butanol, 4-(Methyl-octyl-amino)- butanol, 4-(Ethyl-propyl-amino)-butanol, 4-(Ethyl-iso-propyl-amino)-butanol, 4-(Ethyl- butyl-amino)-butanol, 4-(Ethyl-hexyl-amino)-butanol, 4-(Ethyl-octyl-amino)-butanol, 2-(N- Piperidinyl)-ethanol, 3-(N-Piperidinyl)-propanol, 4-(N-Piperidinyl)-butanol, 5-(N- Piperidinyl)-pentanol, 6-(N-Piperidinyl)-hexanol, 8-(N-Piperidinyl)-octanol, 10-(N- Piperidinyl)-decanol, 12-(N-Piperidinyl)-dodecanol, 2-(N-Pyrrolidinyl)-ethanol, 3-(N-Pyrrolidinyl)-propanol, 4-(N-Pyrrolidinyl)-butanol, 5-(N-,Pyrrolidinyl)-pentyI-, 6-(N-Pyrrolidinyl)-hexanol, 8-(N-Pyrrolidinyl)-octanol, 10-(N-Pyrrolidinyl)-decanol, 12-(N-Pyrrolidinyl)-dodecanol, 2-(N-Morpholino)-ethanol, 3-(N-Morpholino)-propanol, 4-(N- Morpholino)-butanol, 5-(N-Morpholino)-pentanol, 6-(N-Morpholino)-hexanol, 8-(N-Morpholino)-octanol, 10-(N-Morpholino)-decanol, 12-(N-Morpholino)-dodecanol, 2-(N'- Methyl-N-Piperazinyl)-ethanol, 3-(N'-Methyl-N-Piperazinyl)-propanol, 4-(N'-Methyl-N-Piperazinyl)-butanol, 5 -(N'-Methyl-N-Piperazinyl)-pentanol, 6-(N'-Methyl-N-Piperazinyl)- hexanol, 8-(N'-Methyl-N-Piperazinyl)-octanol, 10-(N'-Methyl-N-Piperazinyl)-decanol, 12-(N'-Methyl-N-Piperazinyl)-dodecanol, 2-(N'-Ethyl-N-Piperazinyl)-ethanol, 3 - (N'-Ethyl- N-Piperazinyl)-propanol, 4-(N'-Ethyl-N-Piperazinyl)-butanol, 5 -(N'-Ethyl-N-Piperazinyl)- pentanol, 6-(N'-Ethyl-N-Piperazinyl)-hexanol, 8-(N'-Ethyl-N-Piperazinyl)-octanol, 10-(N'- Ethyl-N-Piperazinyl)-decanol, 12-(N'-Ethyl-N-Piperazinyl)-dodecanol, 2-(N'-iso-Propyl-N- Piperazinyl)-ethanol, 3-(N'-isoPropyl-N-Piperazinyl)-propanol, 4-(N'-iso-Propyl-N-Piperazinyl)-butanol, 5-(N'-iso-Propyl-N-Piperazinyl)-pentanol, 6-(N'-iso-Propyl-N- Piperazinyl)-hexanol, 8-(N'-iso-Propyl-N-Piperazinyl)-octanol, 10-(N'-iso-Propyl-N- Piperazinyi)-decanol, 12-(N'-iso-Propyl-N-Piperazinyl)-dodecanol, 3-Oxa-butanol, 3-Oxa- pentanol, 2,2-Dimethyl-4-oxa-pentanol, 3,6-Dioxa-heptanol, 3,6-Dioxa-octanol, 3,6,9- Trioxa-decanol, 3,6,9-Trioxaundecanol, 4-Oxa-pentanol, 4-Oxa-hexanol, 4-Oxa-heptanol, 4,8-Dioxa-nonanol, 4,8-Dioxa-decanol, 4,8-Dioxa-undecanol, 5-Oxa-hexanol oder 5,10- Dioxa-undecanol.

Weiterhin können ethoxylierte und/oder propoxylierte Alkohole sowie gemischt- ethoxylierte/propoxylierte Alkohole als Lösungsmittel eingesetzt werden, insbesondere R^{a}-(O-CH₂-CH₂)ₓ-OH oder R^{a}-(O-CH(CH₃)-CH₂)ₓ-OH, beziehungsweise R^{a}-(O-CH₂-CH(CH₃))ₓ-OH, worin R^{a} für C₁ bis C₂₀-Alkyl und x für eine ganze Zahl zwischen 10 und 20 steht, oder ethoxylierte und/oder propoxylierte Aminoalkohole, wie zum Beispiel R^{b}₂N(-CH₂-CH₂-O)_{y}-H oder R^{b}₂N(-CH(CH₃)-CH₂-O)_{y}-H beziehungsweise R^{b}₂N(-CH₂CH(CH₃)-O)_{y}-H, worin y für eine ganze Zahl zwischen 1 und 4 steht. R^{b} steht für eine Alkylgruppe mit 1 - 6 Kohlenstoffatomen, wobei der Stickstoff mit den Substituenten R^{b} auch einen fünf- bis siebengliedrigen Ring bilden kann. Der Ring kann gegebenenfalls noch durch eine oder mehrere kurzkettige Alkylgruppen, wie beispielsweise Methyl, Ethyl oder Propyl, substituiert sein. Vorteilhaft kann es jedoch sein, die Methacrylsäure vor der Veresterung noch aufzureinigen, wobei zur Aufreinigung grundsätzlich jedes dem Fachmann bekannte Aufreinigungsverfahren angewendet werden kann, welches herkömmlicher Weise zur Aufreinigung verunreinigter, durch katalytische Gasphasenoxidation von Propylen erhaltener (Meth)Acrylsäure Anwendung findet.

Wurde die Dehydratisierung in der Gasphase durchgeführt, so ist es bevorzugt, dass die Methacrylsäure zunächst unter Erhalt einer wässrigen Methacrylsäure-Lösung kondensiert wird. Dabei kann grundsätzlich jedes dem Fachmann bekannte Kondensationsverfahren eingesetzt werden, beispielsweise eine fraktionierte Kondensation, wie sie in WO-A-2004/035514, WO-A-03/014172 oder EP-A-EP 1 163 201 beschrieben ist, oder durch eine Totalkondensation, wie sie in der EP-A-0 695 736 beschrieben ist. Denkbar ist auch, bei der Kondensation zusätzliches Lösungsmittel, insbesondere Wasser, zuzusetzen, um die Methacrylsäure möglichst vollständig zu absorbieren.

Die nach der Kondensation erhaltene, wässrige Methacrylsäure-Lösung oder aber die im Falle der Flüssigphasendehydratisierung erhaltene wässrige Methacrylsäure-Lösung kann dann in weiteren Aufreinigungsschritten von Wasser und anderen Verunreinigungen befreit werden. Dabei kann zunächst das Wasser in Gegenwart eines Schleppmittels durch Azeotropdestillation entfernt werden, wie dies beispielsweise in der DE-A-198 53 064 beschrieben ist. Denkbar ist auch der Einsatz hochsiedender organischer Lösungsmittel zur Absorption der Methacrylsäure, wie dies beispielsweise in der EP-A-0 974 574 offenbart ist. Neben diesen destillativen Verfahren können auch Membranen zur Entwässerung eingesetzt werden, wie dies etwa in DE-A-44 01 405 vorgeschlagen wird. Denkbar ist weiterhin eine Aufreinigung der im Falle der Flüssigphasendehydratisierung gewonnen oder durch Kondensation erhaltenen wässrigen Methacrylsäure-Lösung durch Kristallisationsverfahren.

Die nach dem Entwässern erhaltene Methacrylsäure kann in weiteren Verfahrensschritten noch weiter aufgereinigt werden. So können noch enthaltene, hochsiedende Verunreinigungen durch weitere Destillationsschritte entfernt werden. Besonders bevorzugt ist es jedoch, wenn die nach dem Entwässern erhaltene Methacrylsäure durch Kristallisationsverfahren weiter aufgereinigt wird, wie dies etwa in DE-A-101 49 353 beschrieben ist.

Die so erhaltene, aufgereinigte Methacrylsäure kann dann gegebenenfalls der Veresterung unterzogen werden.

Einen Beitrag zur Lösung der eingangs genannten Aufgaben leistet weiterhin ein Verfahren zur Herstellung von Methacrylsäure oder Methacrylsäureestern, umfassend die Verfahrensschritte:
IIA) Herstellung von 2-Hydroxyisobuttersäure-Monomereinheiten enthaltenden Polyhydroxyalkanoaten durch das vorstehend beschriebene Verfahren,
IIB) Spaltung der 2-Hydroxyisobuttersäure-Monomereinheiten enthaltenden Polyhydroxyalkanoaten unter Bildung von 2-Hydroxyisobuttersäure sowie gegebenenfalls Neutralisation der 2-Hydroxyisobuttersäure und/oder Aufreinigung der 2-Hydroxyisobuttersäure,
IIC) Dehydratisierung der 2-Hydroxyisobuttersäure unter Bildung von Methacrylsäure sowie gegebenenfalls Veresterung des Methacrylates bzw. der Methacrylsäure.

Einen Beitrag zur Lösung der eingangs genannten Aufgaben leistet auch ein Verfahren zur Herstellung von Polymethacrylsäure oder Polymethacrylsäureestern, umfassend die Verfahrensschritte
IIIA) Herstellung von Methacrylsäure durch das vorstehend beschriebene Verfahren,
IIIB) radikalische Polymerisation der Methacrylsäure,
wobei gegebenenfalls die Carboxylgruppen der Methacrylsäure vor oder nach der radikalischen Polymerisation zumindest teilweise verestert werden können.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Folgende Figuren sind Bestandteil der Beispiele:
Figur 1: Hybridplasmid pET101/D-TOPO::icmA-icmB
Figur 2: Hybridplasmid pBBR1MCS-2::icmA-icmB
Figur 3: Die Quantifizerung von 2-Hydroxyisobuttersäure in der Probe IM-86 erfolgte nach Dotierung der Probe mit 2-Hydroxyisobuttersäure. Der Methyllactat-Peak (Rt. 7,16 min) wurde dabei als interner Standard benutzt. Abgebildet sind die GC-MS-Chromatogramm-Ausschnitte der dotierten und der Original-Probe.
Figur 4: Aufstockung von 2-Hydroxyisobuttersäure zu der Probe IM-89. Abgebildet sind Ausschnitte der NMR-Spektren Probe der Original-Probe (A) und der dotierten Probe (B).

### Beispiele

### 1. Isolierung genomischer DNA und Amplifikation der Fragmente icmA und icmB

Aus dem Stamm *Aquincola tertiaricarbonis* (*A. tertiaricarbonis* DSMZ 18512) wurde mit dem Kit DNeasy Blood & Tissue (Qiagen GmbH, Hilden) nach Herstellerangaben genomische-DNA isoliert und als Matrize für eine PCR zur Amplifikation der Fragmente *icmA* (1,7 kbp; DQ436456) und *icmB* (0,4 kbp; DQ436457) eingesetzt. Diese kodieren für ein Enzym E₃. Dabei wurden die Oligonucleotide Aqt-icmA_fw 5'-CACCATGACCTGGCTTGAGCCGCAG-3' (forward primer; Start-Codon ist unterstrichen) und Aqt-icmA-Hind_rev 5'-AAAAAAGCTTCCTGCTCAGAAGACCGGCGTCTCGCG-3' (reverse primer; Stopp-Codon und HindIII-Schnittstelle sind unterstrichen) zur Amplifikation von *icmA* und die Oligonucleotide Aqt-icmB-Hind_fw 5'-AAAAAAGCTTCCCACCATGGACCAAATCCCGATCCGC-3' (forward primer; Start-Codon und HindIII-Schnittstelle sind unterstrichen) und Aqt-icmB_rev 5'-TCAGCGGGCGCCGCGCGCGGCGAC-3' (reverse primer; Stopp-Codon ist unterstrichen) zur Amplifikation von *icmB* eingesetzt.

Die Polymerasekettenreaktion (PCR, nach SAIKI et al., 1985, Enzymatic amplification of β-globin genomic sequences and restriction site analysis for diagnosis of sickle cell anemia. Science 230:1350-1354.) wurde mit der Pfu-Polymerase (Promega, Madison, USA) angesetzt. Dabei wurden 35 Zyklen mit jeweils 60 Sekunden bei 95°C, 30 Sekunden bei 65°C und 4 Minuten bei 72°C durchgeführt. Die Durchführung der PCR erfolgte in einem Thermocycler (Primus 96 advanced; PEQLAB Biotechnologie GMBH, Erlangen).

Die Fragmente wurden mit dem QIAquick PCR Purification Kit (Qiagen GmbH, Hilden) nach Herstellerangaben aufgereinigt und im Anschluss daran mit HindIII restringiert. Beide Ansätze wurden über die HindIII-Schnittstelle ligiert.

Das Ligationsprodukt *icmA-icmB* (2,1 kbp) wurde als template für eine Pfu-PCR mit den Oligonucleotiden Aqt-icmA_fw 5'-CACCATGACCTGGCTTGAGCCGCAG-3' (forward primer; Start-Codon ist unterstrichen) und Aqt-icmB_rev 5'-TCAGCGGGCGCCGCGCGCGGCGAC-3' (reverse primer; Stop-Codon ist unterstrichen) eingesetzt (35 Zyklen mit jeweils 60 Sekunden bei 95°C, 30 Sekunden bei 65°C und 4,5 Minuten bei 72°C). Das erhaltene PCR-Produkt der entsprechenden Größe wurde mit dem QIAquick PCR Purification Kit (Qiagen GmbH, Hilden) nach Herstellerangaben aufgereinigt.

### 2. Herstellung eines Ralstonia eutropha Expressionsvektors

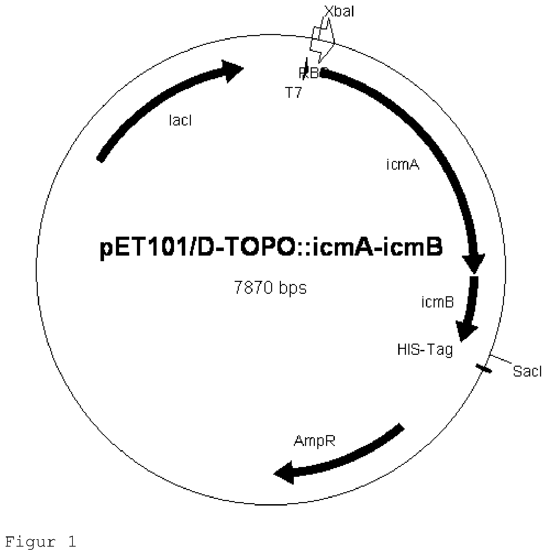

Das aufgereinigte PCR-Fragment *icmA-icmB* (2,1 kbp) wurde nach Angaben des Herstellers in den Vektor pET101/D-TOPO ligiert (Invitrogen GmbH, Karlsruhe). Das erhaltene Hybridplasmid pET101/D-TOPO::*icmA-icmB* (Figur 1, Seq. ID No. 1) wurde in kompetente *E. coli* DH5α-Zellen (New England Biolabs, Frankfurt) transferiert und durch Restriktion und Sequenzierung kontrolliert.

Um eine Expression in *R*. eutropha-Stämmen, welche als Wildtyp Aktivitäten der Enzyme E₁, E₂ und E₄ aufweisen; zu erreichen, musste das Konstrukt in einen geeigneten broad-host-range Vektor kloniert werden. Bei dem benutzten Vektor handelt es sich um pBBR1MCS-2, beschrieben bei KOVACH et al. (1995). Four new derivatives of the broad-host-range cloning vector pBBR1MCS carrying different antibiotic-resistance cassettes. Gene, 166:175-176.

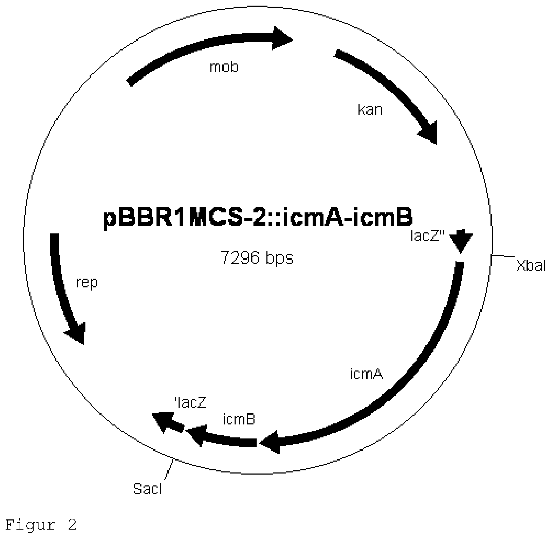

Dazu wurden die Plasmide pET101/D-TOPO::*icmA-icmB* und pBBR1MCS-2 mit den Enzymen XbaI und SacI restringiert und das Fragment *icmA-icmB* in den Zielvektor pBBR1MCS-2 ligiert und kompetente *E*. *coli* DH5α-Zellen (New England Biolabs, Frankfurt) mit dem entstandenen Hybridplasmid pBBR1MCS-2::*icmA-icmB* (Figur 2, Seq. ID No. 2) transformiert.

Das Plasmid wurde durch Restriktion und Sequenzierung überprüft und in kompetente *E. coli* S17-1-Zellen transferiert, ein Stamm, mit dem die konjugative Übertragung von Plasmiden u.a. in *Ralstonia* eutropha-Stämme möglich ist. Dazu wurde eine Spotmating-Konjugation (wie bei FRIEDRICH et al., 1981, Naturally occurring genetic transfer of hydrogen-oxidizing ability between strains of Alcaligenes eutrophus. J Bacteriol 147:198-205 beschrieben) mit *E. coli* S17-1 pBBR1MCS-2::*icmA-icmB* als Donor und *R. eutropha* H16 (reklassifiziert als *Cupriavidus necator,* DSMZ 428) sowie *R. eutropha* PHB-4 (reklassifiziert als *Cupriavidus necator,* DSMZ 541) als Rezipienten durchgeführt.

Es konnten Transkonjuganten erhalten werden, die das Plasmid pBBR1MCS-2::*icmA-icmB* tragen.

### 3. Produktion von 2-Hydroxyisobuttersäure in rekombinanten R. eutropha-Zellen

Die in Beispiel 2 beschriebenen plasmidtragenden *R. eutropha-*Stämme wurden zur Untersuchungen der Bildung von 2-Hydroxyisobuttersäure in 50 mL Vollbrecht-MSM-Medium ((NH₄)₂HPO₄, 2.0 g; KH₂PO₄, 2.1g; MgSO₄ × 7 H20, 0.2 g; FeCl₃ × 6 H₂0, 6 mg; CaCl₂ x 2 H₂0, 10 mg; Spurenelement-Lösung (Pfennig and Lippert, 1966), 0.1 mL). Das Medium wurde zusätzlich mit Na-Gluconat (15 g/L), Kanamycin (50 µg/mL) und Coenzym B12 (60 µg/mL) supplementiert. Die Zellen wurden auf einem temperierbaren Schüttler bei 30 °C und 160 rpm inkubiert. Nach 30 h wurden Na-Gluconat (1,5 %, w/v) und Coenzym B12 [60 µg/mL] nachgefüttert. Die Ernte erfolgte nach 52 h Kultivierung durch Zentrifugation bei 5000 rpm (4°C). Der Kulturüberstand wurde bis zur Analyse bei -20°C gelagert.

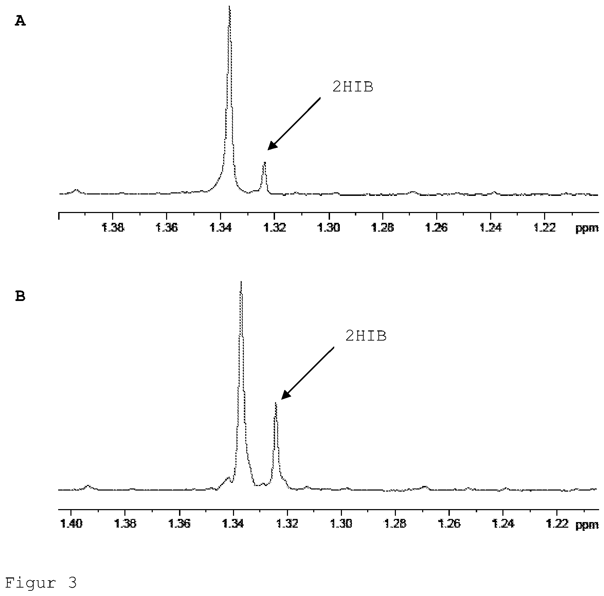

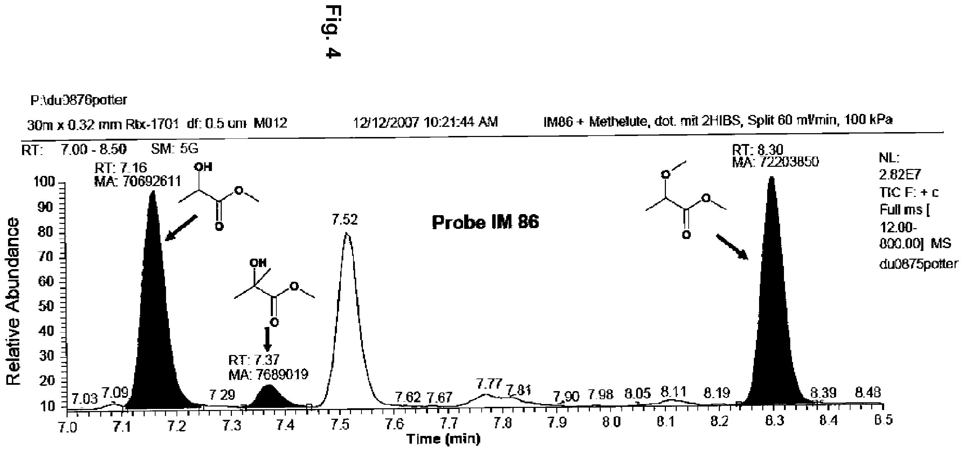

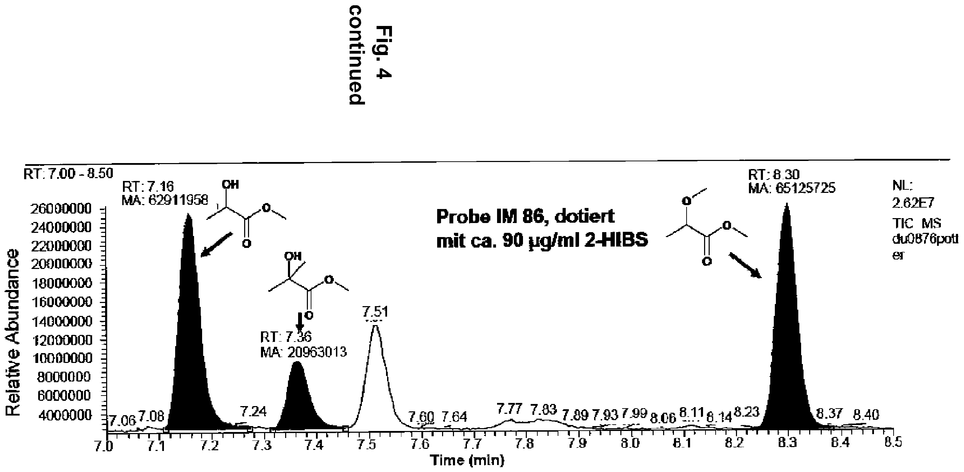

Nachweis und Quantifizierung von 2-Hydroxyisobuttersäure erfolgten mittels quantitativer ¹H-NMR-Spektroskopie. Die Proben wurden quantitativ eingeengt. Von dem Rückstand wurden 1H-NMR-Spektren aufgenommen und der Gehalt gegen TSP (Trisilyl Propionic acid) als internen Standard verrechnet. 2-Hydroxyisobuttersäure zeigt im Spektrum bei ca. 1,36 ppm ein Singulett und wurde zur Absicherung mit der Reinsubstanz aufgestockt. (Figur. 3)
In den analysierten Proben wurde eine Konzentration von maximal 0,72 mmol/kg 2-Hydroxyisobuttersäure detektiert. In entsprechenden Kontrollansätzen mit Leerplasmid wurde dagegen kein 2-Hydroxyisobuttersäure nachgewiesen. Die NMR-Messungen wurden quantitativ und qualitativ mittels GC-MS und Aufstockung mit der Reinsubstanz 2-Hydroxyisobuttersäure bestätigt (Figur 4). Die chromatografische Trennung erfolgte in diesem Fall auf einer 30 m Rtx-1701 Kapillarsäule (Fisher Scientific, Pittsburgh, USA). Die Proben wurden nach der Lyophylisierung mit dem Derivatisierungsreagenz "Methelute" (Pierce, Rockford, USA) aufgenommen. 0,5 µL dieser Lösung wurden direkt über einen Split-/splitless-Injektor aufgegeben. Die Identifizierung des 2-Hydroxyisobuttersäure-Peaks erfolgte durch den Abgleich des Massenspektrums mit Datenbank-Spektren. Zur Abschätzung des Gehaltes an 2-Hydroxyisobuttersäure erfolgte eine Dotierung der Proben mit einer definierten Menge der Vergleichssubstanz 2-Hydroxyisobuttersäure. In den analysierten Proben wurden Konzentrationen von maximal 44 µg/mL detektiert. In Kontrollansätzen mit Leerplasmid wurde kein 2-Hydroxyisobuttersäure nachgewiesen. Analog konnte 2-Hydroxyisobuttersäure, synthetisiert ausgehend von Fructose als C-Quelle (1,5 %, w/v) nachgewiesen werden.

### 4. Dehydratisierung von 2-Hydroxyisobuttersäure zu Methacrylat

5 ml Lösung von 2-Hydroxyisobuttersäure (0,2 g/L), produziert nach Beispiel 3 wird unter Rühren mit NaOH (0.06 mg) versetzt. Die Lösung wird unter Rührung und Rückflusskühlung bei 185-195 °C unter Vakuum (300 torr) inkubiert. Über einen Zeitraum von 5 h wird stündlich jeweils weitere 0,5 mg 2-Hydroxyisobuttersäure in 5 mL zugesetzt, diese enthält 0,4 Gewichtsprozent p-Methoxyphenol, um ein Polymerisieren von Methacrylat zu verhindern. Nach 24 h Inkubation wird die Reaktion beendet. Der Umsatz von 2-Hydroxyisobuttersäure zu Methacrylat beläuft sich auf über 90 %. Die Abtrennung von Methacrylsäure aus dem Reaktionsansatz erfolgt durch Destillation.

## Patentansprüche

1. Eine Bakterienzelle, welche derart gentechnisch verändert wurde, dass sie im Vergleich zu ihrem Wildtyp mehr 2-Hydroxyisobuttersäure zu bilden vermag, **dadurch gekennzeichnet, dass** die Bildung von 2-Hydroxyisobuttersäure über Acetoacetyl-Coenzym A als Zwischenprodukt und 3-Hydroxybutyryl-Coenzym A als Vorprodukt erfolgt, wobei die Zelle mindestens eine Aktivität eines Enzyms E₃ aufweist, welches die Umsetzung von 3-Hydroxybutyryl-Coenzym A zu 2-Hydroxyisobutyryl-Coenzym A katalysiert, wobei die Zelle eine im Vergleich zu ihrem Wildtyp erhöhte Aktivität des Enzyms E₃ aufweist, wobei die Zelle als Wildtyp eine Aktivität eines Enzyms E₄ aufweist, welches die Umsetung von 3-Hydroxybutyryl-Coenzym A zu Polyhydroxybutyrat katalysiert, wobei die Zelle eine im Vergleich zu ihrem Wildtyp erniedrigte Aktivität des Enzyms E₄ aufweist und wobei das Enzym E₃ ausgewählt wird aus einer Hydroxyl-Isobutyryl-CoA Mutase, einer Isobutyryl-CoA Mutase (EC 5.4.99.13) oder einer Methylmalonyl-CoA Mutase (EC 5.4.99.2), und
wobei das Enzym E₄ eine Polyhydroxyalkanoat Synthase ist.

2. Eine Zelle gemäß Anspruch 1, wobei die Zelle als Wildtyp eine Aktivität eines Enzyms E₅ aufweist, welches die Umsetzung von 3-Hydroxybutyryl-Coenzym A zu Crotonyl-Coenzym A katalysiert, wobei dem Enzym E₅ handelt es sich vorzugsweise um eine Crotonase (EC-Number 4.2.1.55) oder um eine (3R)-3-Hydroxybutanoyl-CoA Dehydratase (EC-Number 4.2.1.17).

3. Eine Zelle gemäß Anspruch 2, wobei die Zelle eine im Vergleich zu ihrem Wildtyp erniedrigte Aktivität des Enzyms E₅ aufweist.

4. Eine Zelle gemäß Anspruch 1 bis 3, wobei die Zelle als Wildtyp eine Aktivität eines Enzyms E₆ aufweist, welches die Umsetzung von R-3-Hydroxybutyryl-Coenzym A zu S-3-Hydroxybutyryl-Coenzym A katalysiert, wobei das Enzym E₆ einer 3-Hydroxybutyryl-CoA Epimerase (EC 5.1.2.3) ist.

5. Eine Zelle gemäß mindestens einem der Ansprüche 1 bis 5, wobei die Zelle mindestens eine im Vergleich zu ihrem Wildtyp erniedrigte Aktivität mindestens eines Enzyms E₇ aufweist, welches 3-Hydroxybutyryl-Coenzym A als Substrat akzeptiert.

6. Verfahren zur Herstellung von 2-Hydroxyisobuttersäure oder von 2-Hydroxyisobuttersäure-Monomereinheiten enthaltende Polyhydroxyalkanoaten, umfassend die Verfahrensschritte:
a) in Kontaktbringen einer Zelle gemäß mindestens einem der Ansprüche 1 bis 5 mit einem Nährmedium beinhaltend eine Kohlenstoffquelle unter Bedingungen, unter denen aus der Kohlenstoffquelle 2-Hydroxyisobuttersäure oder 2-Hydroxyisobuttersäure-Monomereinheiten enthaltende Polyhydroxyalkanoate gebildet werden, sowie gegebenenfalls
b) Aufreinigung der 2-Hydroxyisobuttersäure aus dem Nährmedium oder 2-Hydroxyisobuttersäure-Monomereinheiten enthaltende Polyhydroxyalkanoate aus den Zellen.

7. Verfahren zur Herstellung von Methacrylsäure oder Methacrylsäureestern, umfassend die Verfahrensschritte:
IA) Herstellung von 2-Hydroxyisobuttersäure durch ein Verfahren gemäß Anspruch 6 sowie gegebenenfalls Aufreinigung und/oder Neutralisation der 2-Hydroxyisobuttersäure,
IB) Dehydratisierung der 2-Hydroxyisobuttersäure unter Bildung von Methacrylsäure sowie gegebenenfalls Veresterung des Methacrylates bzw. der Methacrylsäure.

8. Verfahren zur Herstellung von Methacrylsäure oder Methacrylsäureestern, umfassend die Verfahrensschritte:
IIA) Herstellung von 2-Hydroxyisobuttersäure-Monomereinheiten enthaltenden Polyhydroxyalkanoaten durch ein Verfahren gemäß Anspruch 6,
IIB) Spaltung der 2-Hydroxyisobuttersäure-Monomereinheiten enthaltenden Polyhydroxyalkanoaten unter Bildung von 2-Hydroxyisobuttersäure sowie gegebenenfalls Neutralisation der 2-Hydroxyisobuttersäure und/oder Aufreinigung der 2-Hydroxyisobuttersäure,
IIC) Dehydratisierung der 2-Hydroxyisobuttersäure unter Bildung von Methacrylsäure sowie gegebenenfalls Veresterung des Methacrylates bzw. der Methacrylsäure.

9. Verfahren zur Herstellung von Polymethacrylsäure oder Polymethacrylsäureestern, umfassend die Verfahrensschritte
IIIA) Herstellung von Methacrylsäure durch ein Verfahren gemäß Anspruch 7 oder 8,
IIIB) radikalische Polymerisation der Methacrylsäure,
wobei gegebenenfalls die Carboxylgruppen der Methacrylsäure vor oder nach der radikalischen Polymerisation zumindest teilweise verestert werden können.

## Claims

1. Bacterial cell which has been genetically modified so as to be capable of producing more 2-hydroxyisobutyric acid than its wild type, **characterized in that** 2-hydroxyisobutyric acid is produced via acetoacetyl-coenzyme A as intermediate and 3-hydroxybutyryl-coenzyme A as precursor, wherein the cell has at least one activity of an enzyme E₃ catalysing the conversion of 3-hydroxybutyryl-coenzyme A to 2-hydroxyisobutyryl-coenzyme A, wherein the cell has a higher enzyme E₃ activity than its wild type, wherein the cell in its wild-type form has an activity of an enzyme E₄ catalysing the conversion of 3-hydroxybutyryl-coenzyme A to polyhydroxybutyrate, wherein the cell has a lower enzyme E₄ activity than its wild type and wherein the enzyme E₃ is selected from a hydroxyl-isobutyryl-CoA mutase, an iabutyryl-CoA mutase (EC 5.4.99.13) or a methylmalonyl-CoA mutase (EC 5.4.99.2), and wherein the enzyme E₄ is a polyhydroxyalkanoate synthase.

2. Cell according to Claim 1, which in its wild-type form has an activity of an enzyme E₅ catalysing the conversion of 3-hydroxybutyryl-coenzyme A to crotonyl coenzyme A, wherein the enzyme E₅ is preferably a crotonase (EC 4.2.1.55) or a (3R)-3-hydroxybutanoyl-CoA dehydratase (EC 4.2.1.17).

3. Cell according to Claim 2, which has a lower enzyme E₅ activity than its wild type.

4. Cell according to Claims 1 to 3, which in its wild-type form has an activity of an enzyme E₆ catalysing the conversion of R-3-hydroxybutyryl-coenzyme A to S-3-hydroxybutyryl-coenzyme A, wherein the enzyme E₆ is a 3-hydroxybutyryl-CoA epimerase (EC 5.1.2.3).

5. Cell according to at least one of Claims 1 to 5, which has at least one lower activity than its wild type of at least one enzyme E₇ accepting 3-hydroxybutyryl-coenzyme A as substrate.

6. Process for preparing 2-hydroxyisobutyric acid or polyhydroxyalkanoates containing 2-hydroxyisobutyric acid monomer units, comprising the steps of:
a) contacting a cell according to at least one of Claims 1 to 5 with a nutrient medium including a carbon source under conditions where 2-hydroxyisobutyric acid or polyhydroxyalkanoates containing 2-hydroxyisobutyric acid monomer units are produced from said carbon source, and where appropriate,
b) purifying the 2-hydroxyisobutyric acid from the nutrient medium or polyhydroxyalkanoates containing 2-hydroxyisobutyric acid monomer units from the cells.

7. Process for preparing methacrylic acid or methacrylic esters, comprising the steps of:
IA) preparing 2-hydroxyisobutyric acid by a process according to Claim 6, and where appropriate purifying and/or neutralizing the 2-hydroxyisobutyric acid,
IB) dehydrating the 2-hydroxyisobutyric acid with production of methacrylic acid and, where appropriate, esterifying the methacrylate or methacrylic acid.

8. Process for preparing methacrylic acid or methacrylic esters, comprising the steps of:
IIA) preparing polyhydroxyalkanoates containing 2-hydroxyisobutyric acid monomer units by a process according to Claim 6,
IIB) cleaving the polyhydroxyalkanoates containing 2-hydroxyisobutyric acid monomer units with production of 2-hydroxyisobutyric acid and, where appropriate, neutralizing the 2-hydroxyisobutyric acid and/or purifying the 2-hydroxyisobutyric acid,
IIC) dehydrating the 2-hydroxyisobutyric acid with production of methacrylic acid and, where appropriate, esterifying the methacrylate or methacrylic acid.

9. Process for preparing poly(methacrylic) acid or poly(methacrylic) esters, comprising the steps of
IIIA) preparing methacrylic acid by a process according to Claim 7 or 8,
IIIB) free-radical polymerization of the methacrylic acid,
wherein, where appropriate, the methacrylic acid carboxyl groups may be esterified at least partially prior to or after the free-radical polymerization.

## Revendications

1. Cellule bactérienne, qui a été modifiée génétiquement de telle sorte qu'elle puisse former davantage d'acide 2-hydroxyisobutyrique en comparaison de son type sauvage, **caractérisée en ce que** la formation d'acide 2-hydroxyisobutyrique a lieu par le biais d'acétoacétyl-coenzyme A en tant que produit intermédiaire et de 3-hydroxybutyryl-coenzyme A en tant que produit préliminaire, la cellule présentant au moins une activité d'une enzyme E₃, qui catalyse la transformation de la 3-hydroxybutyryl-coenzyme A en la 2-hydroxyisobutyryl-coenzyme A, la cellule présentant une activité plus élevée de l'enzyme E₃ en comparaison de son type sauvage, la cellule présentant en tant que type sauvage une activité d'une enzyme E₄, qui catalyse la transformation de la 3-hydroxybutyryl-coenzyme A en polyhydroxybutyrate, la cellule présentant une activité plus faible de l'enzyme E₄ en comparaison de son type sauvage, et l'enzyme E₃ étant choisie parmi une hydroxylisobutyryl-CoA mutase, une isobutyryl-CoA mutase (CE 5.4.99.13) ou une méthylmalonyl-CoA mutase (CE 5.4.99.2) et
l'enzyme E₄ étant une polyhydroxyalcanoate synthase.

2. Cellule selon la revendication 1, dans laquelle la cellule présente en tant que type sauvage une activité d'une enzyme E₅, qui catalyse la transformation de la 3-hydroxybutyryl-coenzyme A en la crotonyl-coenzyme A, l'enzyme E₅ étant de préférence une crotonase (numéro CE-4.2.1.55) ou une (3R)-3-hydroxybutanoyl-CoA déshydratase (numéro CE 4.2.1.17).

3. Cellule selon la revendication 2, dans laquelle la cellule présente une activité plus faible de l'enzyme E₅ en comparaison de son type sauvage.

4. Cellule selon les revendications 1 à 3, dans laquelle la cellule présente en tant que type sauvage une activité d'une enzyme E₆, qui catalyse la transformation de la R-3-hydroxybutyryl-coenzyme A en la S-3-hydroxybutyryl-coenzyme A, l'enzyme E₆ étant une 3-hydroxybutyryl-CoA épimérase (CE 5.1.2.3).

5. Cellule selon au moins l'une quelconque des revendications 1 à 5, la cellule présentant au moins une activité plus faible d'au moins une enzyme E₇, qui accepte la 3-hydroxybutyryl-coenzyme A en tant que substrat, en comparaison de son type sauvage.

6. Procédé de fabrication d'acide 2-hydroxyisobutyrique ou de polyhydroxyalcanoates contenant des unités monomères acide 2-hydroxyisobutyrique, comprenant les étapes de procédé suivantes :
a) la mise en contact d'une cellule selon au moins l'une quelconque des revendications 1 à 5 avec un milieu nutritif contenant une source de carbone dans des conditions dans lesquelles de l'acide 2-hydroxyisobutyrique ou des polyhydroxyalcanoates contenant des unités monomères acide 2-hydroxyisobutyrique sont formés à partir de la source de carbone, et éventuellement
b) la purification de l'acide 2-hydroxyisobutyrique à partir du milieu nutritif ou des polyhydroxyalcanoates contenant des unités monomères acide 2-hydroxyisobutyrique à partir des cellules.

7. Procédé de fabrication d'acide méthacrylique ou d'esters de l'acide méthacrylique, comprenant les étapes de procédé suivantes :
IA) la fabrication d'acide 2-hydroxyisobutyrique par un procédé selon la revendication 6, et éventuellement la purification et/ou la neutralisation de l'acide 2-hydroxyisobutyrique,
IB) la déshydratation de l'acide 2-hydroxyisobutyrique avec formation d'acide méthacrylique, et éventuellement l'estérification du méthacrylate ou de l'acide méthacrylique.

8. Procédé de fabrication d'acide méthacrylique ou d'esters de l'acide méthacrylique, comprenant les étapes de procédé suivantes :
IIA) la fabrication de polyhydroxyalcanoate contenant des unités monomères acide 2-hydroxyisobutyrique par un procédé selon la revendication 6,
IIB) le clivage des polyhydroxyalcanoate contenant des unités monomères acide 2-hydroxyisobutyrique avec formation d'acide 2-hydroxyisobutyrique, et éventuellement la neutralisation de l'acide 2-hydroxyisobutyrique et/ou la purification de l'acide 2-hydroxyisobutyrique,
IIC) la déshydratation de l'acide 2-hydroxyisobutyrique avec formation d'acide méthacrylique, et éventuellement l'estérification du méthacrylate ou de l'acide méthacrylique.

9. Procédé de fabrication d'acide polyméthacrylique ou d'esters de l'acide polyméthacrylique, comprenant les étapes de procédé suivantes :
IIIA) la fabrication d'acide méthacrylique par un procédé selon la revendication 7 ou 8,
IIIB) la polymérisation radicalaire de l'acide méthacrylique,
les groupes carboxyle de l'acide méthacrylique pouvant éventuellement être au moins partiellement estérifiés avant ou après la polymérisation radicalaire.
